(19) Europäisches Patentamt / European Patent Office / Office européen des brevets

(11) EP 4 768 495 A1

(12) **EUROPEAN PATENT APPLICATION**
published in accordance with Art. 153(4) EPC

(43) Date of publication:
**01.07.2026 Bulletin 2026/27**

(21) Application number: **25845710.0**

(22) Date of filing: **03.09.2025**

(51) International Patent Classification (IPC):
***C07J 41/00*** (2006.01) ***A61K 31/57*** (2006.01)
***A61P 25/00*** (2006.01) ***A61P 25/04*** (2006.01)
***A61P 25/06*** (2006.01) ***A61P 25/08*** (2006.01)
***A61P 25/20*** (2006.01) ***A61P 25/22*** (2006.01)
***A61P 25/24*** (2006.01) ***A61P 25/28*** (2006.01)
***A61P 25/16*** (2006.01)

(86) International application number:
**PCT/CN2025/118667**

(87) International publication number:
**WO 2026/091851 (07.05.2026 Gazette 2026/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **31.10.2024 CN 202411536243**

(71) Applicant: **Hunan Mingrui Pharmaceutical Co., Ltd**
**Changsha, Hunan 410329 (CN)**

(72) Inventors:
- **AN, Ming**
  **Changsha, Hunan 410329 (CN)**
- **ZHANG, Xu**
  **Changsha, Hunan 410329 (CN)**
- **SU, Sheng**
  **Changsha, Hunan 410329 (CN)**
- **WANG, Yan**
  **Changsha, Hunan 410329 (CN)**
- **KUANG, Bin**
  **Changsha, Hunan 410329 (CN)**

(74) Representative: **Calysta NV**
**Lambroekstraat 5a**
**1831 Diegem (BE)**

(54) **CRYSTAL FORM OF NEUROACTIVE STEROID DERIVATIVE AND USE THEREOF**

(57) Provided are a crystal form of a neuroactive steroid derivative and use thereof, belonging to the field of pharmaceutical technology. The crystal form of the neuroactive steroid derivative (having a structure represented by formula I) provided by the present disclosure has characteristic diffraction peaks expressed as 2θ angles in an X-ray powder diffraction pattern obtained by using Cu-Kα radiation, including 11.450 ± 0.200°, 15.724 ± 0.200°, 19.040 ± 0.200°, 22.955 ± 0.200° and 24.975 ± 0.200°. The crystal form provided by the present disclosure exhibits good flowability, no electrostatic phenomenon, and high stability, and is almost non-hygroscopic, and can remain stable under high temperature, high humidity and light irradiation conditions. The crystal form has a strong positive modulatory effect on GABAA receptors, has good pharmacokinetic properties and oral absorption rate, and thus can be used for preparation of a drug in oral or other dosage forms for efficiently treating diseases such as postpartum depression.

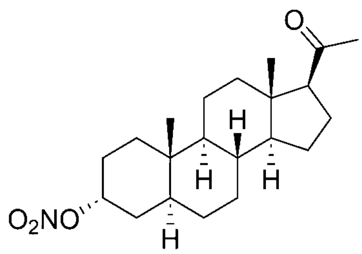

formula I.

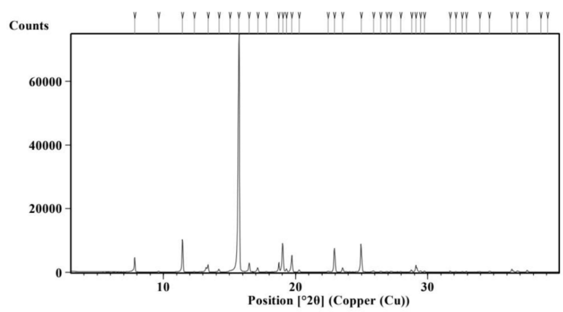


FIG. 2

EP 4 768 495 A1

## Description

The present application claims priority to the Chinese Patent Application No. CN202411536243.7 filed with the China National Intellectual Property Administration on October 31, 2024 and entitled "CRYSTAL FORM OF NEUROACTIVE STEROID DERIVATIVE AND USE THEREOF", which is incorporated herein by reference in its entirety.

## TECHNICAL FIELD

The present disclosure relates to the field of pharmaceutical technology, and in particular to a crystal form of a neuroactive steroid derivative and use thereof.

## BACKGROUND

Allopregnanolone is an endogenous neuroactive steroid (NAS), which is synthesized from progesterone in the brain. NASs primarily interact with the ion channels of γ-aminobutyric acid type A receptors ($GABA_A$ receptors) to achieve a rapid effect on neuronal excitability. NASs can act as positive allosteric modulators (PAMs) of synaptic and extrasynaptic $GABA_A$ receptors, which activate and enhance the phasic current and the tonic current, respectively, thereby ultimately potentiating the tonic inhibitory currents mediated by $GABA_A$ receptors. Allopregnanolone differs from other NASs in that, in addition to activating $GABA_A$ receptors containing α and β subunits, it can also modulate $GABA_A$ receptors containing γ or δ subunits.

Brexanolone is an endogenous neuroactive steroid with a CAS registry number of 516-54-1. On March 19, 2019, the U.S. FDA approved brexanolone (trade name: Zulresso) injection for the treatment of postpartum depression in adult women. This is the first drug specifically targeted at postpartum depression (PPD), which can enhance GABA-mediated currents in mammalian cells expressing recombinant $GABA_A$ receptors. However, the route of administration of brexanolone is continuous intravenous infusion for above 60 h. During the two and a half days of brexanolone infusion, healthcare professionals must provide on-site continuous monitoring of patients, which presents challenges in terms of clinical convenience.

The neuroactive steroid derivative having the structure represented by formula I is a 3-hydroxyl nitrate ester of brexanolone, which has high oral bioavailability while maintaining $GABA_A$ receptor activity, and thus can avoid the inconvenience caused to patients by long-time intravenous infusion.

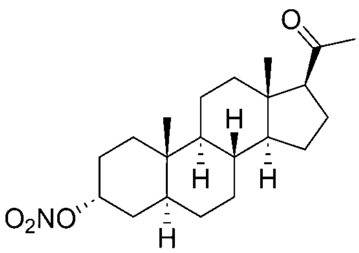
formula I.

However, the above neuroactive steroid derivative having the structure represented by formula I in the related art exhibits poor flowability, which causes inconvenience in the weighing, transfer and use of the product.

## SUMMARY

An object of the present disclosure is to provide a crystal form of a neuroactive steroid derivative and use thereof. The crystal form of the neuroactive steroid derivative provided by the present disclosure exhibits good flowability, which makes the weighing, transfer and use of the product more convenient.

In order to achieve the above object of the present disclosure, the present disclosure provides the following technical solutions.

The present disclosure provides a crystal form of a neuroactive steroid derivative, having characteristic diffraction peaks expressed as 2θ angles in an X-ray powder diffraction pattern obtained by using Cu-Kα radiation, including 11.450 ± 0.200°, 15.724 ± 0.200°, 19.040 ± 0.200°, 22.955 ± 0.200°, and 24.975 ± 0.200°;

where the neuroactive steroid derivative has a structure represented by formula I:

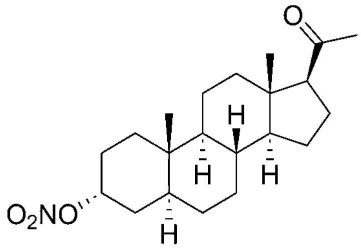

formula I.

In some embodiments, the characteristic diffraction peaks further include one or more selected from the group consisting of: expressed as 2θ angles, 7.843 ± 0.200°, 9.643 ± 0.200°, 12.338 ± 0.200°, 13.384 ± 0.200°, 14.210 ± 0.200°, 15.032 ± 0.200°, 16.498 ± 0.200°, 17.148 ± 0.200°, 17.802 ± 0.200°, 18.739 ± 0.200°, 19.313 ± 0.200°, 19.726 ± 0.200°, 20.276 ± 0.200°, 22.464 ± 0.200°, 23.558 ± 0.200°, 25.897 ± 0.200°, 26.445 ± 0.200°, 26.914 ± 0.200°, 27.202 ± 0.200°, 27.964 ± 0.200°, 28.781 ± 0.200°, 29.102 ± 0.200°, 29.451 ± 0.200°, 29.760 ± 0.200°, 31.706 ± 0.200°, 32.133 ± 0.200°, 32.616 ± 0.200°, 32.922 ± 0.200°, 33.952 ± 0.200°, 34.662 ± 0.200°, 36.355 ± 0.200°, 36.778 ± 0.200°, 37.511 ± 0.200°, 38.559 ± 0.200°, and 39.073 ± 0.200°.

In some embodiments, in the X-ray powder diffraction pattern, there are the characteristic diffraction peaks at 2θ angles: 7.843 ± 0.200°, 11.450 ± 0.200°, 15.724 ± 0.200°, 18.739 ± 0.200°, 19.040 ± 0.200°, 19.726 ± 0.200°, 22.955 ± 0.200°, and 24.975 ± 0.200°.

In some embodiments, in the X-ray powder diffraction pattern, there are the characteristic diffraction peaks at 2θ angles: 7.843 ± 0.200°, 11.450 ± 0.200°, 13.384 ± 0.200°, 15.724 ± 0.200°, 16.498 ± 0.200°, 17.148 ± 0.200°, 18.739 ± 0.200°, 19.040 ± 0.200°, 19.726 ± 0.200°, 20.276 ± 0.200°, 22.955 ± 0.200°, 23.558 ± 0.200°, 24.975 ± 0.200°, 28.781 ± 0.200°, and 29.102 ± 0.200°.

In some embodiments, in the X-ray powder diffraction pattern, there are the characteristic diffraction peaks at 2θ angles: 7.843 ± 0.200°, 11.450 ± 0.200°, 13.384 ± 0.200°, 14.210 ± 0.200°, 15.724 ± 0.200°, 16.498 ± 0.200°, 17.148 ± 0.200°, 18.739 ± 0.200°, 19.040 ± 0.200°, 19.313 ± 0.200°, 19.726 ± 0.200°, 22.955 ± 0.200°, 23.558 ± 0.200°, 24.975 ± 0.200°, 29.102 ± 0.200°, 36.355 ± 0.200°, and 37.511 ± 0.200°.

In some embodiments, in the X-ray powder diffraction pattern, there are the characteristic diffraction peaks at 20 angles: 7.843 ± 0.200°, 11.450 ± 0.200°, 13.384 ± 0.200°, 14.210 ± 0.200°, 15.724 ± 0.200°, 16.498 ± 0.200°, 17.148 ± 0.200°, 18.739 ± 0.200°, 19.040 ± 0.200°, 19.313 ± 0.200°, 19.726 ± 0.200°, 20.276 ± 0.200°, 22.955 ± 0.200°, 23.558 ± 0.200°, 24.975 ± 0.200°, 28.781 ± 0.200°, 29.102 ± 0.200°, 36.355 ± 0.200°, and 37.511 ± 0.200°.

In some embodiments, in the X-ray powder diffraction pattern, there are the characteristic diffraction peaks at 20 angles: 7.843 ± 0.200°, 9.643 ± 0.200°, 11.450 ± 0.200°, 12.338 ± 0.200°, 13.384 ± 0.200°, 14.210 ± 0.200°, 15.032 ± 0.200°, 15.724 ± 0.200°, 16.498 ± 0.200°, 17.148 ± 0.200°, 17.802 ± 0.200°, 18.739 ± 0.200°, 19.040 ± 0.200°, 19.313 ± 0.200°, 19.726 ± 0.200°, 20.276 ± 0.200°, 22.464 ± 0.200°, 22.955 ± 0.200°, 23.558 ± 0.200°, 24.975 ± 0.200°, 25.897 ± 0.200°, 26.445 ± 0.200°, 26.914 ± 0.200°, 27.202 ± 0.200°, 27.964 ± 0.200°, 28.781 ± 0.200°, 29.102 ± 0.200°, 29.451 ± 0.200°, 29.760 ± 0.200°, 31.706 ± 0.200°, 32.133 ± 0.200°, 32.616 ± 0.200°, 32.922 ± 0.200°, 33.952 ± 0.200°, 34.662 ± 0.200°, 36.355 ± 0.200°, 36.778 ± 0.200°, 37.511 ± 0.200°, 38.559 ± 0.200°, and 39.073 ± 0.200°.

In some embodiments, a differential scanning calorimetry pattern of the crystal form of the neuroactive steroid derivative has an endothermic peak at 139.9 °C ± 3 °C; and an onset value of the absorption peak is at 138.5 °C ± 3 °C.

In some embodiments, a thermogravimetric analysis pattern of the crystal form of the neuroactive steroid derivative shows a weight loss of ± 0.5% at 159.4 °C ± 3 °C.

In some embodiments, the crystal form of the neuroactive steroid derivative is of an orthorhombic crystal system, with a space group of $P2_12_12_1$, and the crystal form of the neuroactive steroid derivative has unit cell parameters as follows: a = 7.40840(10)Å, b = 14.3545(2)Å, c = 18.1883(3)Å, α = 90°, β = 90°, γ = 90°; a unit cell volume of 1934.21(5)$Å^3$; and Z = 4.

In some embodiments, the crystal form of the neuroactive steroid derivative has a particle size D90 of 240-530 μm, a D50 of 105-270 μm, and a D10 of 20-65 μm.

In some embodiments, the crystal form of the neuroactive steroid derivative has an angle of repose of 39.2° and a Carr index of 14%.

The present disclosure provides use of the crystal form of the neuroactive steroid derivative according to the above technical solutions in preparation of a drug for treating emotional disorders, psychiatric diseases, neurodegenerative diseases, or neuropathic pain.

In some embodiments, the emotional disorders include generalized anxiety disorder, panic disorder, obsessive-compulsive disorder, post-traumatic stress disorder, social anxiety disorder, premenstrual dysphoric disorder, major depression, or postpartum depression.

In some embodiments, the psychiatric diseases include epilepsy, convulsion, or insomnia.

In some embodiments, the neurodegenerative diseases include Alzheimer's disease, Parkinson's disease, multiple sclerosis, or Niemann-Pick disease type C.

In some embodiments, the neuropathic pain includes menstrual and postmenopausal migraine, diabetic peripheral neuralgia, chemotherapy-induced pain, or sciatica.

In some embodiments, the drug includes an active ingredient and a pharmaceutically acceptable carrier, where the active ingredient is the crystal form of the neuroactive steroid derivative; and the pharmaceutically acceptable carrier is a solid carrier or a liquid carrier.

In some embodiments, the solid carrier includes one or more selected from the group consisting of cellulose, glucose, lactose, mannitol, magnesium stearate, magnesium carbonate, sodium carbonate, sodium saccharin, sucrose, dextrin, talc, starch, pectin, gelatin, tragacanth gum, acacia gum, sodium alginate, paraben, methylcellulose, sodium carboxymethylcellulose, low-melting wax, and cocoa butter.

In some embodiments, the liquid carrier includes one or more selected from the group consisting of water, ethanol, a polyol, a vegetable oil, glyceride, agar, pyrogen-free water, isotonic saline and Ringer's solution.

In some embodiments, the polyol includes one or more selected from the group consisting of glycerol, propylene glycol and liquid polyethylene glycol; the vegetable oil includes one or more of peanut oil, cottonseed oil, safflower oil, sesame oil, olive oil, corn oil and soybean oil.

In some embodiments, an administration route of the drug includes oral administration, intrapulmonary administration, intranasal administration, or transdermal administration.

In some embodiments, a dosage form of the drug includes a tablet, a lozenge, a buccal tablet, a suspension, a dispersible powder, a dispersible granule, an emulsion, a hard capsule, a soft capsule, a syrup, an elixir, a solution, a spray, an aerosol or dry powder formulation, a nasal drop or a nasal spray.

The present disclosure provides a crystal form (designated as crystal form A) of a neuroactive steroid derivative (having a structure represented by formula I, designated as a compound of formula I), having characteristic diffraction peaks expressed as $2\theta$ angles in an X-ray powder diffraction pattern obtained using Cu-K$\alpha$ radiation, including 11.450 $\pm$ 0.200°, 15.724 $\pm$ 0.200°, 19.040 $\pm$ 0.200°, 22.955 $\pm$ 0.200°, and 24.975 $\pm$ 0.200°. Compared with the amorphous form of the compound of formula I, the crystal form A of the compound of formula I of the present disclosure has a smaller angle of repose and Carr index, indicating that the crystal form A of the compound of formula I of the present disclosure exhibits better flowability, which makes the weighing, transfer and use of the product more convenient, such as the effective reduction in time for mixing with an excipient.

Furthermore, the crystal form A of the compound of formula I provided by the present disclosure exhibits no electrostatic phenomenon, and high stability, and is almost non-hygroscopic, and can remain stable under high temperature, high humidity and light irradiation conditions without significant degradation, which meets the requirements for storage and subsequent processing and production. This avoids the risk of crystal transformation during storage and processing, which could otherwise lead to changes in the quality of the pharmaceutical formulation and further cause issues with clinical efficacy. The crystal form A of the compound of formula I provided by the present disclosure has a strong positive modulatory effect on $GABA_A$ receptors, has good pharmacokinetic (PK) properties and oral absorption rate, and thus can be used for the preparation of a drug in oral or other dosage forms for efficiently treating diseases such as postpartum depression.

## BRIEF DESCRIPTION OF THE DRAWINGS

FIG. 1 shows an X-ray powder diffraction (XRPD) pattern of the amorphous form of the compound of formula I in comparative example of the present disclosure;

FIG. 2 shows an XRPD pattern of the crystal form A of the compound of formula I in example of the present disclosure;

FIG. 3 shows a differential scanning calorimetry (DSC) pattern of the crystal form A of the compound of formula I in example of the present disclosure;

FIG. 4 shows a thermogravimetric analysis (TGA) pattern of the crystal form A of the compound of formula I in example of the present disclosure;

FIG. 5 shows a particle size distribution diagram of the crystal form A of the compound of formula I in example of the present disclosure;

FIG. 6 shows a dynamic vapor sorption (DVS) analysis pattern of the crystal form A of the compound of formula I in example of the present disclosure;

FIG. 7 shows an ellipsoid plot of the three-dimensional structure of the compound of formula I;

FIG. 8 shows a comparison diagram of the half-maximal effective concentrations of the crystal form A of the compound of formula I in example of the present disclosure and allopregnanolone against the $GABA_A$ receptor; and

FIG. 9 shows the mean plasma drug concentration-time curves in male and female SD rats after single gavage of 10 mg/kg, 30 mg/kg, and 100 mg/kg of crystal form A of the compound of formula I in test examples of the present disclosure.

## DETAILED DESCRIPTION OF THE EMBODIMENTS

The present disclosure provides a crystal form of a neuroactive steroid derivative, having characteristic diffraction peaks expressed as 20 angles in an X-ray powder diffraction pattern obtained using Cu-Kα radiation, including 11.450 ± 0.200°, 15.724 ± 0.200°, 19.040 ± 0.200°, 22.955 ± 0.200° and 24.975 ± 0.200°;
where the neuroactive steroid derivative has a structure represented by formula I:

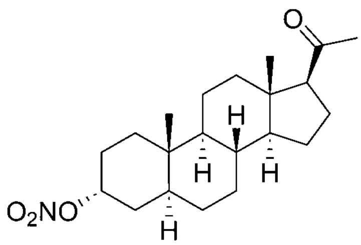
formula I.

In embodiments of the present disclosure, the characteristic diffraction peaks may also include one or more of: expressed as 20 angles, 7.843 ± 0.200°, 9.643 ± 0.200°, 12.338 ± 0.200°, 13.384 ± 0.200°, 14.210 ± 0.200°, 15.032 ± 0.200°, 16.498 ± 0.200°, 17.148 ± 0.200°, 17.802 ± 0.200°, 18.739 ± 0.200°, 19.313 ± 0.200°, 19.726 ± 0.200°, 20.276 ± 0.200°, 22.464 ± 0.200°, 23.558 ± 0.200°, 25.897 ± 0.200°, 26.445 ± 0.200°, 26.914 ± 0.200°, 27.202 ± 0.200°, 27.964 ± 0.200°, 28.781 ± 0.200°, 29.102 ± 0.200°, 29.451 ± 0.200°, 29.760 ± 0.200°, 31.706 ± 0.200°, **32.133** ± 0.200°, 32.616 ± 0.200°, 32.922 ± 0.200°, 33.952 ± 0.200°, 34.662 ± 0.200°, 36.355 ± 0.200°, 36.778 ± 0.200°, 37.511 ± 0.200°, 38.559 ± 0.200°, and 39.073 ± 0.200°.

In embodiments of the present disclosure, in the X-ray powder diffraction pattern obtained using Cu-Kα radiation of the crystal form of the neuroactive steroid derivative, there are the characteristic diffraction peaks at 20 angles: 7.843 ± 0.200°, 11.450 ± 0.200°, 15.724 ± 0.200°, 18.739 ± 0.200°, 19.040 ± 0.200°, 19.726 ± 0.200°, 22.955 ± 0.200°, and 24.975 ± 0.200°.

In embodiments of the present disclosure, in the X-ray powder diffraction pattern obtained using Cu-Kα radiation of the crystal form of the neuroactive steroid derivative, there are the characteristic diffraction peaks at 2θ angles: 7.843 ± 0.200°, 11.450 ± 0.200°, 13.384 ± 0.200°, 15.724 ± 0.200°, 16.498 ± 0.200°, 17.148 ± 0.200°, 18.739 ± 0.200°, 19.040 ± 0.200°, 19.726 ± 0.200°, 20.276 ± 0.200°, 22.955 ± 0.200°, 23.558 ± 0.200°, 24.975 ± 0.200°, 28.781 ± 0.200°, and 29.102 ± 0.200°.

In embodiments of the present disclosure, in the X-ray powder diffraction pattern obtained using Cu-Kα radiation of the crystal form of the neuroactive steroid derivative, there are the characteristic diffraction peaks at 20 angles: 7.843 ± 0.200°, 11.450 ± 0.200°, 13.384 ± 0.200°, 14.210 ± 0.200°, 15.724 ± 0.200°, 16.498 ± 0.200°, 17.148 ± 0.200°, 18.739 ± 0.200°, 19.040 ± 0.200°, 19.313 ± 0.200°, 19.726 ± 0.200°, 22.955 ± 0.200°, 23.558 ± 0.200°, 24.975 ± 0.200°, 29.102 ± 0.200°, 36.355 ± 0.200°, and 37.511 ± 0.200°.

In embodiments of the present disclosure, in the X-ray powder diffraction pattern obtained using Cu-Kα radiation of the crystal form of the neuroactive steroid derivative, there are the characteristic diffraction peaks at 2θ angles: 7.843 ± 0.200°, 11.450 ± 0.200°, 13.384 ± 0.200°, 14.210 ± 0.200°, 15.724 ± 0.200°, 16.498 ± 0.200°, 17.148 ± 0.200°, 18.739 ± 0.200°, 19.040 ± 0.200°, 19.313 ± 0.200°, 19.726 ± 0.200°, 20.276 ± 0.200°, 22.955 ± 0.200°, 23.558 ± 0.200°, 24.975 ± 0.200°, 28.781 ± 0.200°, 29.102 ± 0.200°, 36.355 ± 0.200°, and 37.511 ± 0.200°.

In embodiments of the present disclosure, in the X-ray powder diffraction pattern obtained by using Cu-Kα radiation of the crystal form of the neuroactive steroid derivative, there are the characteristic diffraction peaks at 20 angles: 7.843 ± 0.200°, 9.643 ± 0.200°, 11.450 ± 0.200°, 12.338 ± 0.200°, 13.384 ± 0.200°, 14.210 ± 0.200°, 15.032 ± 0.200°, 15.724 ± 0.200°, 16.498 ± 0.200°, 17.148 ± 0.200°, 17.802 ± 0.200°, 18.739 ± 0.200°, 19.040 ± 0.200°, 19.313 ± 0.200°, 19.726 ± 0.200°, 20.276 ± 0.200°, 22.464 ± 0.200°, 22.955 ± 0.200°, 23.558 ± 0.200°, 24.975 ± 0.200°, 25.897 ± 0.200°, 26.445 ± 0.200°, 26.914 ± 0.200°, 27.202 ± 0.200°, 27.964 ± 0.200°, 28.781 ± 0.200°, 29.102 ± 0.200°, 29.451 ± 0.200°, 29.760 ± 0.200°, 31.706 ± 0.200°, 32.133 ± 0.200°, 32.616 ± 0.200°, 32.922 ± 0.200°, 33.952 ± 0.200°, 34.662 ± 0.200°, 36.355 ± 0.200°, 36.778 ± 0.200°, 37.511 ± 0.200°, 38.559 ± 0.200°, and 39.073 ± 0.200°.

In embodiments of the present disclosure, the X-ray powder diffraction pattern obtained by using Cu-Kα radiation of the crystal form of the neuroactive steroid derivative is shown in FIG. 2, with an error within ± 0.20°. In the present disclosure, the analytical data corresponding to FIG. 2 is shown in Table 1.

**Table 1 Analytical data for XRPD pattern of crystal form A of the compound of formula I**

| No. | 2θ angle (°) | Interplanar spacing (Å) | Peak height | Relative intensity (%) | No. | 2θ angle (°) | Interplanar spacing (Å) | Peak height | Relative intensity (%) |
|---|---|---|---|---|---|---|---|---|---|
| 1 | 7.843 | 11.27 | 4612 | 6.10 | 21 | 25.897 | 3.44 | 359 | 0.47 |
| 2 | 9.643 | 9.17 | 250 | 0.33 | 22 | 26.445 | 3.37 | 260 | 0.34 |
| 3 | 11.450 | 7.73 | 10265 | 13.58 | 23 | 26.914 | 3.31 | 103 | 0.14 |
| 4 | 12.338 | 7.17 | 94 | 0.12 | 24 | 27.202 | 3.28 | 214 | 0.28 |
| 5 | 13.384 | 6.62 | 2299 | 3.04 | 25 | 27.964 | 3.19 | 163 | 0.22 |
| 6 | 14.210 | 6.23 | 847 | 1.12 | 26 | 28.781 | 3.10 | 736 | 0.97 |
| 7 | 15.032 | 5.89 | 437 | 0.58 | 27 | 29.102 | 3.07 | 1914 | 2.53 |
| 8 | 15.724 | 5.64 | 75581 | 100 | 28 | 29.451 | 3.03 | 396 | 0.52 |
| 9 | 16.498 | 5.37 | 2872 | 3.80 | 29 | 29.760 | 3.00 | 352 | 0.47 |
| 10 | 17.148 | 5.17 | 1290 | 1.71 | 30 | 31.706 | 2.82 | 319 | 0.42 |
| 11 | 17.802 | 4.98 | 156 | 0.21 | 31 | 32.133 | 2.79 | 132 | 0.17 |
| 12 | 18.739 | 4.74 | 3046 | 4.03 | 32 | 32.616 | 2.75 | 144 | 0.19 |
| 13 | 19.040 | 4.66 | 8976 | 11.88 | 33 | 32.922 | 2.72 | 201 | 0.27 |
| 14 | 19.313 | 4.60 | 988 | 1.31 | 34 | 33.952 | 2.64 | 123 | 0.16 |
| 15 | 19.726 | 4.50 | 5327 | 7.05 | 35 | 34.662 | 2.59 | 264 | 0.35 |
| 16 | 20.276 | 4.38 | 681 | 0.90 | 36 | 36.355 | 2.47 | 839 | 1.11 |
| 17 | 22.464 | 3.96 | 279 | 0.37 | 37 | 36.778 | 2.44 | 344 | 0.46 |
| 18 | 22.955 | 3.87 | 7399 | 9.79 | 38 | 37.511 | 2.40 | 563 | 0.75 |
| 19 | 23.558 | 3.78 | 1333 | 1.76 | 39 | 38.559 | 2.33 | 45 | 0.06 |
| 20 | 24.975 | 3.57 | 8067 | 10.67 | 40 | 39.073 | 2.31 | 13 | 0.02 |

In embodiments of the present disclosure, in the differential scanning calorimetry pattern of the crystal form of the neuroactive steroid derivative, there is an endothermic peak specifically at 139.9 °C ± 3 °C; in the differential scanning calorimetry pattern of the crystal form of the neuroactive steroid derivative, specifically an onset value of the endothermic peak is at 138.5 °C ± 3 °C. In the embodiments of the present disclosure, the differential scanning calorimetry pattern of the crystal form of the neuroactive steroid derivative is shown in FIG. 3 (unless otherwise specified, the exothermic peak in the DSC pattern points upward), in which the onset temperature is 40.0 °C, the heating rate is 10.00 °C/min, the temperature is held at 170.0 °C, with a hold time of 0 min; the endothermic peak is at 139.93 °C, the extrapolated onset is at 138.52 °C, and the extrapolated endset is at 142.49 °C; the Calorie is -72.03 J/g.

In embodiments of the present disclosure, the thermogravimetric analysis pattern of the crystal form of the neuroactive steroid derivative shows a weight loss of ± 0.5% at 159.4 °C ± 3 °C, which, for example, is specifically -0.1%. In the embodiments of the present disclosure, the thermogravimetric analysis pattern of the crystal form of the neuroactive steroid derivative is shown in FIG. 4, in which the heating rate is 10.00 °C/min, the temperature is held at 300.0 °C, with a hold time of 0 min; the time at onset is 7.62 min, at a temperature of 90.00 °C; the time at endset is 14.62 min, at a temperature 160.00 °C; the time at midpoint is 0.00 min, at a temperature of 0.00 °C; the extrapolated onset temperature is 90.08 °C and the extrapolated endset temperature is 159.39 °C; the weight loss is -0.100%.

In embodiments of the present disclosure, the crystal form of the neuroactive steroid derivative is specifically of an orthorhombic crystal system, with a space group being specifically $P2_12_12_1$; the unit cell parameters are specifically as follows: a = 7.40840(10)Å, b = 14.3545(2)Å, c = 18.1883(3)Å, α = 90°, β = 90°, γ = 90°; a unit cell volume of 1934.21(5)$Å^3$; and Z = 4. In the present disclosure, the relevant crystal data for the crystal form of the neuroactive steroid derivative are detailed in Table 5 of the test examples, the atomic coordinates and equivalent isotropic displacement parameters are detailed in Table 6 of the test examples, the bond lengths are detailed in Table 7 of the test examples, the bond angles are detailed in Table 8 of the test examples, and the torsion angles are detailed in Table 9 of the test examples, which will not be repeated here.

In embodiments of the present disclosure, the crystal form of the neuroactive steroid derivative has a particle size

D90 of 240-530 μm, a D50 of 105-270 μm, and a D10 of 20-65 μm; specifically, the crystal form of the neuroactive steroid derivative has a particle size D90 of 240-380 μm, a D50 of 105-180 μm, and a D10 of 20-45 μm.

In embodiments of the present disclosure, the crystal form of the neuroactive steroid derivative has an angle of repose of 39.2° and a Carr index of 14%, indicating that the crystal form exhibits good flowability.

The present disclosure provides a method for preparing the crystal form of the neuroactive steroid derivative described in the above technical solutions, which includes steps of:

mixing a neuroactive steroid derivative having a structure represented by formula I with an organic solvent, and dissolving the neuroactive steroid derivative by heating to obtain a dissolved solution; and

cooling the dissolved solution to a temperature of 0-10 °C at a rate of 18-22 °C/h, maintaining the temperature for crystallization, and performing solid-liquid separation and drying a resulting solid to obtain the crystal form of the neuroactive steroid derivative.

In the present disclosure, unless otherwise specified, the raw materials and reagents used are commercially available products well known to those skilled in the art or are prepared by methods well known to those skilled in the art; the organic solvents used are directly commercially available products and can be used without further purification.

In the present disclosure, a neuroactive steroid derivative having a structure represented by formula I (i.e., a compound of formula I) is mixed with an organic solvent, and dissolved by heating to obtain a dissolved solution. As an embodiment of the present disclosure, the organic solvent includes absolute ethanol, ethyl acetate, acetone, n-heptane, methyl tert-butyl ether, or dimethyl sulfoxide, which may specifically be absolute ethanol. As an embodiment of the present disclosure, an amount ratio of the compound of formula I to the organic solvent is 98 g: 0.8-1.2 L, specifically 98 g: 0.9-1 L. As an embodiment of the present disclosure, dissolving by heating is performed at a temperature of 50-110 °C, and the temperature may specifically be selected according to the type of organic solvent. For example, when the organic solvent is absolute ethanol, the temperature for dissolving by heating may be 75-85 °C, which may specifically be 78-80 °C; when the organic solvents are ethyl acetate, acetone, n-heptane, methyl tert-butyl ether, and dimethyl sulfoxide, respectively, the temperature for dissolving by heating may be 70-80 °C, 50-60 °C, 90-100 °C, 50-60 °C and 100-110 °C, respectively. In some embodiments of the present disclosure, dissolving by heating is performed under stirring conditions; after dissolving by heating, the stirring is continued for 25-35 min (which may specifically be 28-30 min) to obtain the dissolved solution.

After obtaining the dissolved solution, in the present disclosure, the dissolved solution is cooled to a temperature of 0-10 °C at a rate of 18-22 °C/h, and maintained at the temperature for crystallization, and solid-liquid separation is performed and a resulting solid is dried to obtain the crystal form of the neuroactive steroid derivative. In the present disclosure, during the cooling and crystallization, the cooling rate is 18-22 °C/h, which may specifically be 19-20 °C/h; in the present disclosure, the cooling is stopped when the temperature is lowered to 0-10 °C (which may specifically be 3-5 °C), the temperature is maintained for crystallization, and solid-liquid separation is performed and a resulting solid is dried to obtain the crystal form of the neuroactive steroid derivative (i.e., the crystal form A of the compound of formula I). As an embodiment of the present disclosure, the time for the crystallization is 25-35 min, which may specifically be 28-30 min; the crystallization may be performed under stirring conditions. As an embodiment of the present disclosure, the solid-liquid separation is performed by filtration. As an embodiment of the present disclosure, drying a resulting solid is performed by vacuum drying the solid obtained from the solid-liquid separation; a vacuum degree for the vacuum drying may be -0.07 to -0.08 MPa, which may specifically be -0.075 to -0.078 MPa; a temperature for the vacuum drying may be 45-55 °C, which may specifically be 48-50 °C; a time for the vacuum drying may be 8-12 h, which may specifically be 9-10 h. The crystal form A of the compound of formula I of the present disclosure is specifically a white crystalline powder.

The present disclosure provides use of the crystal form of the neuroactive steroid derivative according to the above technical solutions or the crystal form of the neuroactive steroid derivative prepared by the method according to the above technical solutions in preparation of a drug for treating emotional disorders, psychiatric diseases, neurodegenerative diseases, or neuropathic pain. As an embodiment of the present disclosure, the emotional disorders include generalized anxiety disorder, panic disorder, obsessive-compulsive disorder, post-traumatic stress disorder, social anxiety disorder, premenstrual dysphoric disorder, major depression, or postpartum depression; the psychiatric diseases include epilepsy, convulsion, or insomnia; the neurodegenerative diseases include Alzheimer's disease, Parkinson's disease, multiple sclerosis, or Niemann-Pick disease type C; the neuropathic pain includes menstrual and postmenopausal migraine, diabetic peripheral neuralgia, chemotherapy-induced pain or sciatica.

In the present disclosure, the drug specifically includes an active ingredient and a pharmaceutically acceptable carrier, where the active ingredient is the crystal form of the neuroactive steroid derivative according to the above technical solutions. As an embodiment of the present disclosure, the pharmaceutically acceptable carrier may be a solid carrier or a liquid carrier; the solid carrier includes, but is not limited to, one or more of cellulose, glucose, lactose, mannitol, magnesium stearate, magnesium carbonate, sodium carbonate, sodium saccharin, sucrose, dextrin, talc, starch, pectin, gelatin, tragacanth gum, acacia gum, sodium alginate, paraben, methylcellulose, sodium carboxymethylcellulose, low-melting wax, and cocoa butter; the liquid carrier includes, but is not limited to, one or more of water, ethanol, a polyol (e.g.,

one or more of glycerol, propylene glycol, and liquid polyethylene glycol), a vegetable oil (e.g., one or more of peanut oil, cottonseed oil, safflower oil, sesame oil, olive oil, corn oil and soybean oil), glyceride, agar, pyrogen-free water, isotonic saline, and Ringer's solution.

In some embodiments of the present disclosure, the dosage form of the drug includes a tablet, a lozenge, a buccal tablet, a suspension (e.g., an aqueous or oily suspension), a dispersible powder, a dispersible granule, an emulsion, a hard capsule, a soft capsule, a syrup, an elixir, a solution, a spray, an aerosol or dry powder formulation, a nasal drop or a nasal spray; the administration route of the drug may include oral administration, intrapulmonary administration, intranasal administration, or transdermal administration.

The crystal form A of the compound of formula I provided by the present disclosure can act as positive allosteric modulators (PAMs) of synaptic and extrasynaptic $GABA_A$ receptors, which activate and enhance the phasic current and the tonic current, respectively, thereby ultimately potentiating the tonic inhibitory currents mediated by $GABA_A$ receptors. The crystal form A of the compound of formula I of the present disclosure has good pharmacokinetic (PK) properties and oral absorption rate, and can be used for the treatment of diseases such as postpartum depression. The crystal form A of the compound of formula I of the present disclosure is stable, and almost non-hygroscopic, is less affected by light and heat, and is less prone to moisture absorption and deterioration during formulation, resulting in a longer storage period for the pharmaceutical product.

The technical solutions in the present disclosure will be described clearly and completely below in conjunction with the examples of the present disclosure. Apparently, the described examples are merely some rather than all of the examples of the present disclosure. Based on the examples of the present disclosure, all other examples that could be obtained by those of ordinary skill in the art without any creative efforts shall fall within the scope of the present disclosure.

Some of the test methods referred to in the following test examples of the present disclosure are as follows:

1. X-ray powder diffraction (XRPD) method

Instrument model: Empyrean X-ray diffractometer.
Test method: About 10-20 mg of a sample was used for XRPD detection.
XRPD detection parameters include:

X-ray tube: Cu, Kα, λ = 1.54060Å;
X-ray tube voltage: 45 kV;
X-ray tube current: 40 mA;
divergence slit: 1/8°;
detector slit: 7.5 mm;
anti-scatter slit: 1/4°;
scan range: 3-40°;
step size: 0.0263°; and
scan time: 46.665 s.

2. Differential scanning calorimetry (DSC) method

Instrument model: DSC-60 Plus Shimadzu differential scanning calorimeter.
Test method: A sample (2-5 mg) was placed in a covered aluminum crucible and tested under the protection of dry nitrogen at a flow rate of 50 mL/min. The procedure was as follows: heating from 40 °C to the set test temperature at a rate of 10 °C/min.

3. Thermal gravimetric analyzer (TGA) method

Instrument model: TGA-50M Shimadzu thermogravimetric analyzer.
Test method: A sample (2-5 mg) was placed in an aluminium oxide crucible and tested under the protection of dry nitrogen at a flow rate of 50 mL/min. The procedure was as follows: increasing temperature from room temperature to 350 °C at a heating rate of 10 °C/min.

4. Dynamic vapor sorption (DVS) method

Instrument model: Quantachrome dynamic vapor sorption analyzer.
Test method: A sample (10-15 mg) was placed in a DVS sample pan for testing.
DVS test parameters included:

temperature: 25 °C;

equilibrium: dm/dt = 0.002%/min (min: 10 min, max: 180 min);
drying: drying at 0% RH for 120 min;
RH (%) testing gradients:

10% (from 0% RH to 90% RH, from 90% RH to 0% RH);
5% (from 90% RH to 95% RH, from 95% RH to 90% RH);
RH (%) testing gradient range: 0% to 95%.

The evaluation categories of hygroscopicity are shown in Table 2:

**Table 2 Evaluation categories of hygroscopicity**

| Categories of hygroscopicity | ΔW% |
|---|---|
| Deliquescent | Absorbing sufficient water to form a solution |
| Extremely hygroscopic | ΔW% ≥ 15% |
| Hygroscopic | 15% > ΔW% ≥ 2% |
| Slightly hygroscopic | 2% > ΔW% ≥ 0.2% |
| Non-hygroscopic or almost non-hygroscopic | ΔW% < 0.2% |
| Note: ΔW% represents the hygroscopic weight gain of the test sample at 25 ± 1 °C and 80 ± 2% RH. | |

5. X-ray single crystal diffraction method

Instrument model: single crystal X-ray diffractometer Rigaku Oxford Diffraction XtaLAB Synergy-S.

Test method: directly performed on the sample provided.
Detector: HyPix-6000HE area detector;
cryogenic system: Oxford Cryostream 800;
light source: Cu: λ = 1.54184Å, 50 W;
distance from crystal to detector: d = 35 mm;
X-ray tube voltage: 50 kV; and
X-ray tube current: 1 mA.

**Comparative Example 1: Preparation of amorphous form sample of compound of formula I**

Dichloromethane (DCM, 42 mL) was added to a 250 mL three-necked flask, and allopregnanolone (2.0 g) was added thereto under stirring. The resulting mixture was then cooled to -10 °C. Acetic anhydride ($Ac_2O$, 18 mL) was added dropwise. After the dropwise addition, the temperature was maintained at -10 °C, and concentrated nitric acid (4 mL, with a concentration of 63-65 wt%) was added dropwise to the system. After the dropwise addition, the reaction was performed under stirring at -10 °C for 3 h. After the reaction was completed, the reaction solution was washed sequentially with purified water, saturated aqueous sodium bicarbonate solution, and purified water, and concentrated under reduced pressure to obtain a crude product, which was purified by column chromatography (eluent: ethyl acetate and petroleum ether, a volume ratio of ethyl acetate to petroleum ether being 1 : 10) to obtain an amorphous form sample of the compound of formula I (1.9 g, with a yield of 83.2%) as an off-white solid.
The XRPD pattern of the amorphous form of the compound of formula I is shown in FIG. 1.

**Example 1: Preparation of crystal form A of compound of formula I**

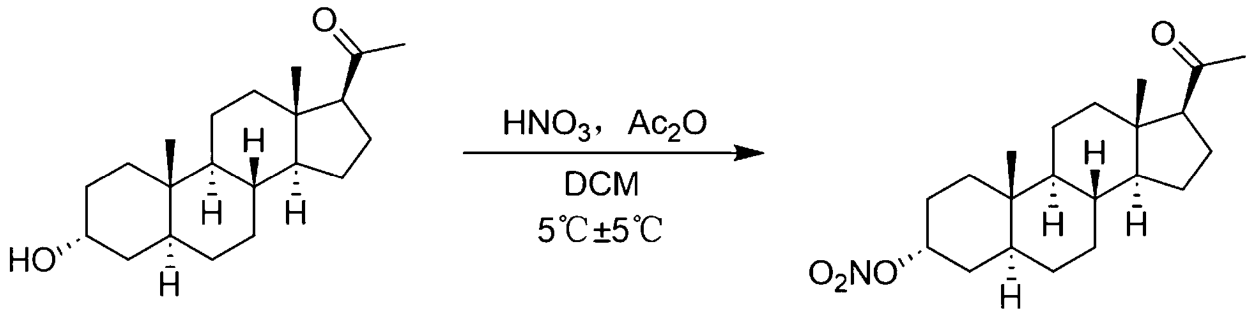


**Allopregnanolone** **I**

Dichloromethane (DCM, 5.0 L) was added to a reaction kettle, and allopregnanolone (490 g) was added thereto under stirring. The resulting mixture was then cooled to 5 ± 5 °C, and acetic anhydride ($Ac_2O$, 1.27 kg) was added dropwise. After the dropwise addition, concentrated nitric acid (780 g, with a concentration of 63-65 wt%) was added dropwise to the system, and the temperature of the system was controlled at 5 ± 5 °C during the dropwise addition. After the dropwise addition, the reaction was performed under stirring for 1.0 ± 0.5 h. After the reaction was completed, the resulting product system was washed sequentially with purified water, saturated aqueous sodium bicarbonate solution, and purified water, and concentrated under reduced pressure to obtain a crude product 1 of the compound of formula I.

Anhydrous ethanol (5.0 L) was added to a reaction kettle, and the crude product 1 of the compound of formula I (568 g) was added under stirring. The mixture was heated to 80 ± 5 °C, and stirring was continued for 30 min. Subsequently, the mixture was cooled to 5 ± 5 °C and filtered, and a resulting filter cake was dried under vacuum at -0.075 MPa and 50 ± 5 °C for 10 ± 2 h to obtain a crude product 2 of the compound of formula I.

Absolute ethanol (1.0 L) was added to a reaction kettle, and the crude product 2 of the compound of formula I (98 g) was added to the reaction kettle under stirring. The mixture was heated to 80 ± 5 °C until the solid was completely dissolved, and stirring was continued for 30 min. Subsequently, a resulting mixture was cooled for crystallization, i.e., cooled to 5 ± 5 °C at a cooling rate of 20 °C/h, and stirring was continued for 30 min. The resulting mixture was filtered, and a resulting filter cake was dried under vacuum at -0.075 MPa and 50 ± 5 °C for 10 ± 2 h to obtain the crystal form A of the compound of formula I (85 g, with a yield of 77.6%) as a white crystalline powder.

$^1H$ NMR (400 MHz, $CDCl_3$): δ 5.23 - 5.17 (m, 1H), 2.53 (t, J = 9.0 Hz, 1H), 2.15 (d, J = 9.7 Hz, 1H), 2.11 (s, 3H), 2.00 (dt, J = 11.8, 3.2 Hz, 1H), 1.97 - 1.89 (m, 1H), 1.78 (dd, J = 14.1, 3.3 Hz, 1H), 1.70 - 1.53 (m, 7H), 1.50 - 1.35 (m, 3H), 1.30 - 1.13 (m, 6H), 0.95 (dd, J = 12.1, 4.8 Hz, 1H), 0.81 (m, 4H), 0.61 (s, 3H).

MS: 364.30 $[M+H]^+$.

The XRPD pattern of the crystal form A of the compound of formula I is shown in FIG. 2, the DSC pattern is shown in FIG. 3, and the TGA pattern is shown in FIG. 4.

FIG. 5 shows a particle size distribution diagram of the crystal form A of the compound of formula I (tested by dry method). The results showed that the particle size D90 was 377 μm, D50 was 176 μm, and D10 was 41.1 μm.

In the following test examples, the crystal form A of the compound of formula I used was the one prepared according to the method described in Example 1, while the amorphous form sample of the compound of formula I used was the one prepared according to the method described in Comparative Example 1.

**Test Example 1: Mechanical stability test of crystal form A of compound of formula I**

In this test example, a mechanical stability test was performed on the crystal form A of the compound of formula I. The results are shown in Table 3. It can be seen that the crystal form of the compound of formula I does not change under the mechanical conditions as shown in Table 3.

**Table 3 Mechanical stability test results for crystal form A of compound of formula I**

| No. | Mechanical condition | Crystal form |
|---|---|---|
| 1 | Mortar grinding for 30 min | Crystal form A |
| 2 | Crushing in mechanical crusher for 30 min | Crystal form A |
| 3 | Tableting with a tablet press | Crystal form A |

**Test Example 2: Hygroscopicity analysis of crystal form A of compound of formula I**

In this test example, dynamic vapor sorption analysis was performed on the crystal form A of the compound of formula I. The DVS pattern is shown in FIG. 6. It can be seen that the crystal form A of the compound of formula I of the present disclosure is almost non-hygroscopic.

**Test Example 3: Solid stability test of crystal form A of compound of formula I**

According to the "Guidelines for the Stability Testing of Drug Substances and Preparations" (Chinese Pharmacopoeia 2020 Edition, Part IV, General Chapter 9001), the stability of the crystal form A of the compound of formula I prepared in Example 1 was investigated under the conditions of high temperature (60 °C, exposed), high humidity (25 °C/92.5% RH, exposed) and strong light irradiation (white light intensity: 5500 Lx; UV light intensity: 95 $\mu W/cm^2$, exposed), specifically as follows:

15 mg of the crystal form A of the compound of formula I was weighed, placed at the bottom of a glass sample vial, and spread into a thin layer. The vials containing the samples placed under high temperature and high humidity conditions were sealed with aluminum foil, and several small holes were provided in the aluminum foil to ensure that the samples were in full contact with the ambient air. The vial containing the sample placed under strong light irradiation was sealed with a threaded cap. The samples placed under different conditions were sampled for testing (XRPD) on day 5, day 10 and month 1. The test results were compared with the initial test results on day 0. The experimental results are shown in Table 4. It can be seen that the crystal form A of the compound of formula I has good crystal form stability under high temperature, high humidity and strong light irradiation conditions.

**Table 4 Solid stability test results for crystal form A of compound of formula I**

| Test conditions | Time point | Crystal form |
|---|---|---|
| - | Day 0 | Crystal form A |
| High temperature (oven at 60 °C, exposed) | Day 5 | Crystal form A |
| | Day 10 | Crystal form A |
| | Month 1 | Crystal form A |
| High humidity (25 °C/92.5% RH, exposed) | Day 5 | Crystal form A |
| | Day 10 | Crystal form A |
| | Month 1 | Crystal form A |
| Strong light irradiation (5500 Lx, exposed) | Day 5 | Crystal form A |
| | Day 10 | Crystal form A |
| | Month 1 | Crystal form A |

**Test Example 4: X-ray single crystal diffraction analysis of crystal form A of compound of formula I**

In this test example, X-ray single crystal diffraction analysis was performed on the crystal form A of the compound of formula I. The analysis results showed that, for the product prepared in Example 1, one unit cell contained one molecule of the compound of formula I. The crystal was detected as a colorless massive crystal ($0.20 \times 0.05 \times 0.05$ $mm^3$), belonging to the orthorhombic crystal system with a $P2_12_12_1$ space group. The unit cell parameters were as follows: a = 7.40840(10) Å, b = 14.3545(2)Å, c = 18.1883(3)Å, $\alpha$ = 90°, $\beta$ = 90°, $\gamma$ = 90°, V = 1934.21(5)$Å^3$, and Z = 4. The calculated density Dc = 1.248 $g/cm^3$, the number of electrons in unit cell F(000) = 792.0, the linear absorption coefficient of unit cell $\mu$(Cu K$\alpha$) = 0.682 $mm^{-1}$, and diffraction experiment temperature T = 150.00(10) K. The specific crystal data is shown in Table 5. The stereospecific configurations of chiral centers of the compound were confirmed by single crystal diffraction analysis as 3R, 5S, 8R, 9S, 10S, 13S, 14S, and 17S. The ellipsoid plot of the molecular structure of the compound of formula I is shown in FIG. 7. The atomic coordinates and equivalent isotropic displacement parameters are shown in Table 6, the bond lengths are shown in Table 7, the bond angles are shown in Table 8, and the torsion angles are shown in Table 9.

**Table 5 Crystal data for single crystal of compound of formula I**

| | |
|---|---|
| Crystal size/$mm^3$ | $0.20 \times 0.05 \times 0.05$ |
| Diffraction radiation source | Cu K$\alpha$ ($\lambda$ = 1.54184Å) |

(continued)

| Crystal system | Orthorhombic crystal system |
|---|---|
| Space group | $P2_12_12_1$ |
| Unit cell parameters a, b, c/Å | 7.40840(10), 14.3545(2), 18.1883(3) |
| Unit cell parameters $\alpha$, $\beta$, $\gamma$/° | 90, 90, 90 |
| Unit cell volume/$Å^3$ | 1934.21(5) |
| Number of molecules per unit cell | 4 |
| Crystal density (calculated) $g/cm^3$ | 1.248 |
| Number of electrons in unit cell | 792.0 |
| Linear absorption coefficient in unit cell $\mu/mm^{-1}$ | 0.682 |
| Diffraction index range | $-8 \leq h \leq 8$, $-17 \leq k \leq 17$, $-21 \leq 1 \leq 21$ |
| Diffraction experiment temperature/K | 150.00(10) |
| 2θ range for data collection/° | 7.846-133.162 |
| Goodness of fit based on $F^2$ | 1.063 |
| Residual factor [$I \geq 2\sigma$ (I)] | $R_1$ = 0.0320, $wR_2$ = 0.0830 |
| Residual factor [all data] | $R_1$ = 0.0324, $wR_2$ = 0.0833 |
| Peak and valley of residual electron density/e $Å^{-3}$ | 0.13/-0.21 |
| Number of diffraction points collected/number of independent diffraction points [Diffraction intensity deviation] | 69549/3420[$R_{int}$ = 0.0790] |
| Flack parameter | -0.01(8) |

**Table 6 Atomic coordinates ($\times 10^4$) and equivalent isotropic displacement parameters ($Å^2 \times 10^3$) of single crystal of compound of formula I**

| Atom | *x* | *y* | *z* | U(eq) |
|---|---|---|---|---|
| O1 | 599.4(18) | 1953.7(9) | 6891.3(7) | 26.1(3) |
| O4 | 9971(2) | 6949.2(10) | 4167.4(9) | 36.4(4) |
| O3 | -633(2) | 683.9(10) | 6485.8(8) | 37.1(4) |
| O2 | 917(2) | 602.1(10) | 7494.7(9) | 40.4(4) |
| N1 | 277(2) | 1003.7(11) | 6971,1(9) | 27.4(4) |
| C16 | 7365(2) | 4406.8(12) | 5121.0(10) | 21.6(4) |
| C5 | 3657(2) | 2215.0(12) | 7372.0(10) | 24.0(4) |
| C7 | 4030(2) | 3782.9(12) | 6709.8(10) | 20.9(4) |
| C15 | 7056(3) | 5471.5(11) | 5072.0(9) | 21.2(4) |
| C20 | 8412(3) | 6678.7(12) | 4171.8(10) | 25.9(4) |
| C20 | 8412(3) | 6678.7(12) | 4171,8(10) | 25.9(4) |
| C8 | 4730(2) | 4196.6(11) | 5969.1(10) | 20.7(4) |
| C19 | 7917(3) | 5667.6(12) | 4298.0(10) | 23.9(4) |
| C6 | 4394(2) | 2717.7(12) | 6692.9(10) | 20.9(4) |
| C9 | 6761(2) | 3988.3(11) | 5851.0(10) | 21.2(4) |
| C13 | 5033(3) | 5652.1(12) | 5146.0(10) | 24.9(4) |
| C2 | 1969(3) | 3902.9(12) | 6774.0(11) | 25.5(4) |
| C12 | 4302(3) | 5241.4(12) | 5869.7(10) | 25.2(4) |

(continued)

| Atom | *x* | *y* | *z* | U(eq) |
|---|---|---|---|---|
| C4 | 1658(3) | 2385.9(12) | 7491.4(10) | 24.1(4) |
| C10 | 7110(3) | 2935.4(12) | 5871.2(11) | 25.1(4) |
| C11 | 6376(3) | 2488.1(12) | 6570.2(11) | 26.6(4) |
| C17 | 9324(3) | 4279.1(12) | 4878.7(10) | 26.5(4) |
| C14 | 8115(3) | 6014.7(13) | 5659.7(10) | 26.5(4) |
| C1 | 4945(3) | 4261.3(13) | 7369.7(11) | 30.2(4) |
| C3 | 1168(3) | 3412.6(13) | 7447.8(11) | 28.1(4) |
| C18 | 9537(3) | 4989.7(13) | 4248.1(11) | 32.6(5) |
| C21 | 6890(3) | 7357.3(14) | 4059.3(12) | 36.1(5) |

**Table 7 Bond length of single crystal of compound of formula I**

| Atom | Atom | Bond length/Å | Atom | Atom | Bond length/Å |
|---|---|---|---|---|---|
| O1 | N1 | 1.392(2) | C15 | C13 | 1.527(3) |
| O1 | C4 | 1.480(2) | C15 | C14 | 1.538(2) |
| O4 | C20 | 1.218(3) | C20 | C19 | 1.514(2) |
| O3 | N1 | 1.202(2) | C20 | C21 | 1.504(3) |
| O2 | N1 | 1.210(2) | C8 | C9 | 1.549(2) |
| C16 | C15 | 1.548(2) | C8 | C12 | 1.543(2) |
| C16 | C9 | 1.525(2) | C19 | C18 | 1.547(3) |
| C16 | C17 | 1.528(3) | C6 | C11 | 1,521(3) |
| C5 | C6 | 1.531(3) | C9 | CI0 | 1.534(2) |
| C5 | C4 | 1,517(3) | C13 | C12 | 1.541(2) |
| C7 | C8 | 1.561(2) | C2 | C3 | 1.533(3) |
| C7 | C6 | 1.553(2) | C4 | C3 | 1.520(2) |
| C7 | C2 | 1.541(2) | C10 | C11 | 1.525(3) |
| C7 | C1 | 1.540(2) | C17 | C18 | 1.543(3) |
| C15 | C19 | 1.571(2) | / | / | / |

**Table 8 Bond angle of single crystal of compound of formula I**

| Atom | Atom | Atom | Bond angle/° | Atom | Atom | Atom | Bond angle/° |
|---|---|---|---|---|---|---|---|
| N1 | O1 | C4 | 115.13(13) | C9 | C8 | C7 | 111.65(14) |
| O3 | N1 | O1 | 113.19(15) | C12 | C8 | C7 | 113.74(14) |
| O3 | N1 | O2 | 128.01(17) | C12 | C8 | C9 | 111.76(14) |
| O2 | N1 | O1 | 118.80(15) | C20 | C19 | C15 | 113.94(14) |
| C9 | C16 | C15 | 113.31(14) | C20 | C19 | C18 | 113.97(17) |
| C9 | C16 | C17 | 118.88(15) | C18 | C19 | C15 | 104.77(14) |
| C17 | C16 | C15 | 104.04(15) | C5 | C6 | C7 | 112.71(15) |
| C4 | C5 | C6 | 112.80(15) | C11 | C6 | C5 | 111.12(15) |
| C6 | C7 | C8 | 107.45(14) | C11 | C6 | C7 | 112.58(14) |
| C2 | C7 | C8 | 110.62(15) | C16 | C9 | C8 | 109.26(14) |

(continued)

| Atom | Atom | Atom | Bond angle/° | Atom | Atom | Atom | Bond angle/° |
|---|---|---|---|---|---|---|---|
| C2 | C7 | C6 | 106.48(14) | C16 | C9 | C10 | 111.07(15) |
| C1 | C7 | C8 | 110.91(14) | C10 | C9 | C8 | 110.55(14) |
| C1 | C7 | C6 | 112.21(15) | C15 | C13 | C12 | 110.80(15) |
| C1 | C7 | C2 | 109.09(15) | C3 | C2 | C7 | 113.13(16) |
| C16 | C15 | C19 | 99.69(13) | C13 | C12 | C8 | 113.57(15) |
| C13 | C15 | C16 | 107.94(14) | 01 | C4 | C5 | 110.11(15) |
| C13 | C15 | C19 | 116.58(15) | 01 | C4 | C3 | 103.97(14) |
| C13 | C15 | C14 | 110.70(15) | CS | C4 | C3 | 112,49(15) |
| C14 | C15 | C16 | 112.63(15) | C11 | C10 | C9 | 112.01(15) |
| C14 | C15 | C19 | 108.93(15) | C6 | C11 | C10 | 112.04(15) |
| O4 | C20 | C19 | 122.39(18) | C16 | C17 | C18 | 103.43(15) |
| O4 | C20 | C21 | 120.22(17) | C4 | C3 | C2 | 113.24(15) |
| C21 | C20 | C19 | 117.38(17) | C17 | C18 | C19 | 107.03(16) |

**Table 9 Torsion angle of single crystal of compound of formula I**

| A | B | C | D | Torsion angle/° | A | B | C | D | Torsion angle/° |
|---|---|---|---|---|---|---|---|---|---|
| O1 | C4 | C3 | C2 | -71.20(19) | C9 | C16 | C15 | C19 | 176.56(14) |
| O4 | C20 | C19 | C15 | 107.4(2) | C9 | C16 | C15 | C13 | -61,27(19) |
| O4 | C20 | C19 | C18 | -12.8(3) | C9 | C16 | C15 | C14 | 61.2(2) |
| N1 | O1 | C4 | C5 | 81.74(18) | C9 | C16 | C17 | C18 | -165.81(15) |
| N1 | O1 | C4 | C3 | -157.53(14) | C9 | C8 | C12 | C13 | 51.1(2) |
| C16 | C15 | C19 | C20 | -160.80(16) | C9 | C10 | C11 | C6 | 53.3(2) |
| C16 | C15 | C19 | C18 | -35.57(18) | C13 | C15 | C19 | C20 | 83.4(2) |
| C16 | C15 | C13 | C12 | 57.00(19) | C13 | C15 | C19 | C18 | -151.35(16) |
| C16 | C9 | C10 | C11 | -175.30(15) | C2 | C7 | C8 | C9 | -173.66(14) |
| C16 | C17 | C18 | C19 | 15.3(2) | C2 | C7 | C8 | C12 | 58.73(19) |
| C5 | C6 | C11 | C10 | 176.63(15) | C2 | C7 | C6 | C5 | -57.80(19) |
| C5 | C4 | C3 | C2 | 47.9(2) | C2 | C7 | C6 | C11 | 175.54(15) |
| C7 | C8 | C9 | C16 | 179.93(13) | C12 | C8 | C9 | C16 | -51.40(19) |
| C7 | C8 | C9 | C10 | 57.39(19) | C12 | C8 | C9 | C10 | -173.94(14) |
| C7 | C8 | C12 | C13 | 178.70(15) | C4 | 01 | N1 | 03 | 177.80(15) |
| C7 | C6 | C11 | C10 | -55.9(2) | C4 | O1 | N1 | O2 | -2.5(2) |
| C7 | C2 | C3 | C4 | -54.1(2) | C4 | C5 | C6 | C7 | 55.5(2) |
| C15 | C16 | C9 | C8 | 58.18(19) | C4 | C5 | C6 | C11 | -177.03(15) |
| C15 | C16 | C9 | C10 | -179.60(15) | C17 | C16 | C15 | C19 | 46.04(17) |
| C15 | C16 | C17 | C18 | -38.68(18) | C17 | C16 | C15 | C13 | 168.20(14) |
| C15 | C19 | C18 | C17 | 13.1(2) | C17 | C16 | C15 | C14 | -69.28(18) |
| C15 | C13 | C12 | C8 | -54.5(2) | C17 | C16 | C9 | C8 | -179.18(15) |
| C20 | C19 | C18 | C17 | 138.26(16) | C17 | C16 | C9 | C10 | -57.0(2) |

(continued)

| A | B | C | D | Torsion angle/° | A | B | C | D | Torsion angle/° |
|---|---|---|---|---|---|---|---|---|---|
| C8 | C7 | C6 | C5 | -176.35(14) | C14 | C15 | C19 | C20 | -42.7(2) |
| C8 | C7 | C6 | C11 | 56.99(19) | C14 | C15 | C19 | C18 | 82.54(18) |
| C8 | C7 | C2 | C3 | 173.43(14) | C14 | C15 | C13 | C12 | -66.69(18) |
| C8 | C9 | C10 | C11 | -53.8(2) | C1 | C7 | C8 | C9 | 65.15(18) |
| C19 | C15 | C13 | C12 | 168.09(14) | C1 | C7 | C8 | C12 | -62.46(19) |
| C6 | C5 | C4 | O1 | 66.76(18) | CI | C7 | C6 | C5 | 61.5(2) |
| C6 | C5 | C4 | C3 | -48.7(2) | CI | C7 | C6 | C11 | -65.2(2) |
| C6 | C7 | C8 | C9 | -57.82(17) | CI | C7 | C2 | C3 | -64.31(19) |
| C6 | C7 | C8 | C12 | 174.57(14) | C21 | C20 | C19 | C15 | -71.8(2) |
| C6 | C7 | C2 | C3 | 56.99(19) | C21 | C20 | C19 | C18 | 168.02(17) |

**Test Example 5: Powder properties of crystal form A of compound of formula I**

50 g of each of an amorphous form sample of the compound of formula I and a crystal form A sample of the compound of formula I were weighed. The angle of repose of the sample was measured using an angle of repose tester. The bulk density and tap density of the sample were measured using a tap density tester, from which the Carr index was calculated. The specific results are shown in Table 10 and Table 11. It can be seen that the crystal form A of the compound of formula I prepared by the present disclosure has a smaller angle of repose than that of the amorphous form sample, and a smaller Carr index than that of the amorphous form sample. This indicates that the crystal form A of the compound of formula I prepared by the present disclosure exhibits better flowability compared with the amorphous form.

**Table 10 Angle of repose of amorphous form and crystal form A of compound of formula I**

| No. | Amorphous form/° | Crystal form A/° |
|---|---|---|
| 1 | 47.2 | 39.0 |
| 2 | 47.1 | 39.0 |
| 3 | 47.4 | 39.5 |
| Mean | 47.2 | 39.2 |

**Table 11 Carr indices for amorphous form and crystal form A of compound of formula I.**

| Item | Amorphous form | | Crystal form A | |
|---|---|---|---|---|
| Weight of 100 mL graduated cylinder (g) | 107.43 | 107.45 | 107.43 | 107.45 |
| Initial weight (g) | 41.15 | 41.21 | 48.18 | 48.60 |
| Initial volume (mL) | 100 | 100 | 100 | 100 |
| Tapped volume (mL) | 79 | 79 | 85 | 85 |
| Bulk density (g/mL) | 0.41 | 0.41 | 0.48 | 0.49 |
| Tap density (g/mL) | 0.52 | 0.52 | 0.57 | 0.57 |
| Average bulk density (g/mL) | 0.41 | | 0.49 | |
| Average tap density (g/mL) | 0.52 | | 0.57 | |
| Carr index (%) | 21 | | 14 | |

**Test Example 6: Electrostatic phenomenon analysis of crystal form A of compound of formula I**

Weighing papers were each folded diagonally and placed on the balance pan. 1 g of each of the amorphous form

sample of the compound of formula I and the crystal form A sample of the compound of formula I was weighed. The weighing papers containing the above samples were picked up, gently squeezed in hand, and then transferred to beakers. The amorphous form sample of the compound of formula I was fluffy, could be easily squeezed into a cake-like shape, and showed significant residue on the weighing paper; the crystal form A sample of the compound of formula I was loose, showed no cake-forming phenomenon after squeezing, and showed no residue on the weighing paper after transfer. It can be seen that compared with the amorphous form sample of the compound of formula I, the crystal form A of the compound of formula I shows no apparent electrostatic phenomenon, is easier to transfer, and exhibits better flowability.

**Test Example 7: Determination of positive modulatory activity of crystal form A of compound of formula I against $GABA_A$ receptor**

1. Experimental method:

In the experiment, a HEK293 cell line transiently expressing the $GABA_A$ ($\alpha4\beta3\delta$) receptor was used to investigate the effect of the inventive sample (i.e., the crystal form A sample of the compound of formula I) on the $GABA_A$ ($\alpha4\beta3\delta$) receptor, and the half-maximal effective concentration ($EC_{50}$) value was tested for the compound. In the experiment, a manual patch-clamp system, including an HEKA EPC 10 signal amplifier and a digital switching system, was used for whole-cell current recording of chloride ion currents in the $GABA_A$ channel. All experiments were performed under normal room temperature conditions.

The $GABA_A$ ($\alpha4\beta3\delta$) receptor current after exposure to the mixture having each concentration of GABA and 3 $\mu$M of the inventive sample, and the current of the control mixture of saturated GABA and 3 $\mu$M of the inventive sample were normalized (Peak $current_{compound}$/Peak $current_{control}$). For each concentration ratio, the mean, standard deviation and standard error were calculated.

$$Y = Bottom + (Top - Bottom)/(1 + 10\hat{}((LogEC_{50} - X) \times HillSlope))$$

The $EC_{50}$ value of the inventive sample was calculated according to the above equation, and the dose-dependent effect was subjected to non-linear fitting, where $EC_{50}$ was the half-maximal effective concentration. The calculation of $EC_{50}$ and curve fitting were performed using GraphPad Prism software.

With unchanged experimental protocol and experimental parameters, the above experiment was repeated with allopregnanolone in replace of the inventive sample. The $EC_{50}$ value of allopregnanolone was calculated according to the above equation, and the dose-dependent effect was subjected to non-linear fitting, where $EC_{50}$ was the half-maximal effective concentration. The calculation of $EC_{50}$ and curve fitting were performed using GraphPad Prism software.

2. Experimental results:

FIG. 8 shows a comparison diagram of the half-maximal effective concentrations ($EC_{50}$) of the crystal form A of the compound of formula I and allopregnanolone for the $GABA_A$ receptor. In FIG. 8, compound 1 represents the crystal form A of the compound of formula I. The relevant data are shown in Table 12. The results show that the half-maximal effective concentration ($EC_{50}$) value of the crystal form A of the compound of formula I against the $GABA_A$ receptor is 10.514 nM, and the half-maximal effective concentration ($EC_{50}$) value of allopregnanolone for the $GABA_A$ receptor is 8.328 nM. This indicates that the inventive sample has a strong positive modulatory effect on the $GABA_A$ receptor, with activity close to that of allopregnanolone.

**Table 12 Half-maximal effect concentrations of crystal form A of compound of formula I and allopregnanolone for $GABA_A$ receptor**

| Item | Crystal form A of compound of formula I (3 $\mu$M) | Allopregnanolone (3 $\mu$M) |
|---|---|---|
| HillSlope | 0.5627 | 0.2059 |
| $EC_{50}$ | 10.514 | 8.328 |

**Test Example 8: Pharmacokinetic study of crystal form A of compound of formula I in male and female SD rats after single intravenous bolus injection and gavage, and 7-day repeated gavage**

1. Experimental purpose: To evaluate the pharmacokinetic behavior of the crystal form A of the compound of formula I after single intravenous bolus injection and gavage, investigate the bioavailability after gavage, compare the differences

between male and female rats, and investigate whether accumulation or induction occurs in rats after seven consecutive days of gavage.

2. Experimental drug:

Preparation of the formulation for intravenous bolus injection:

The vehicle was a 30% (w/v) sulfobutyl-β-cyclodextrin (SBE-β-CD) aqueous solution, and the preparation was carried out according to the following procedures:

a. About 50% by volume of purified water was added to a suitable container and heated to 80-90 °C.
b. When the temperature of the water reached a specified value range, the heater was turned off, and the required amount of sulfobutyl-β-cyclodextrin was slowly added to the above container with continuous stirring.
c. The stirring was continued until the temperature dropped to room temperature and a clear solution was visually confirmed.
d. An appropriate amount of the crystal form A of the compound of formula I was added to the above solution, and the volume of a resulting solution was made up to the final volume with an appropriate amount of purified water.
e. The resulting solution was continuously stirred and ultrasonically mixed at 40 °C until a homogeneous and clear solution was formed.
f. The above solution was sterilized by filtration through a 0.22 μm microporous filter membrane and stored at room temperature for later use.

Preparation of the formulation for gavage:

The vehicle was sesame oil and the preparation was performed according to the following procedures:

1. An appropriate amount of the test sample was weighed into a suitable container, and an appropriate volume of the vehicle was slowly added to the container.
2. Continuous stirring was performed until a homogeneous formulation was visually confirmed.
3. The volume of a resulting solution was made up to the final volume with the vehicle, and continuous stirring was performed until a homogeneous formulation was visually confirmed.

The prepared formulation was stored in a refrigerator at 2-8 °C away from light and used on the same day.

3. Study method and experimental design:

After the animals (specifically as shown in Table 13) arrived at the animal facility, they were acclimated for at least 3 days. At the end of the acclimation period, the veterinarian or designated person examined the health status of the animals to assess whether the animals were suitable for the test study.

**Table 13 Relevant information of experimental animals**

| | |
|---|---|
| Species | Rat |
| Strain | SD |
| Source | Beijing Vital River Laboratory Animal Technology Co., Ltd., China |
| Gender/number | 30 rats (15 males and 15 females) |
| Week of age at the start of the test | 7-10 weeks old |
| Body weight at the start of the test | 200-400 g (male); 150-300 g (female) |

Before the first administration, the animals were divided into five groups based on their body weight. The test design is summarized in Table 14.

**Table 14 Test design for intravenous bolus injection and gavage of crystal form A of compound of formula I**

| Group # | Gender/number | | Treatment | | | | Route of administration | Frequency of administration |
|---|---|---|---|---|---|---|---|---|
| | Male | Female | Dose (mg/kg) | Concentration (mg/mL) | Volume (mL/kg) | Vehicle | | |
| 1 | 3 | 3 | 3 | 0.3 | 10 | 30% (w/v) SBE-β-CD aqueous solution | Intravenous bolus injection | Once |
| 2 | 3 | 3 | 10 | 2 | 5 | Sesame oil | Gavage | Once |
| 3 | 3 | 3 | 30 | 6 | 5 | | Gavage | Once |
| 4 | 3 | 3 | 100 | 20 | 5 | | Gavage | Once |
| 5 | 3 | 3 | 30 | 6 | 5 | | Gavage | Once daily for seven consecutive days |
| Animals in groups 1, 2, 3 and 4 received a single administration on Day 1 only; animals in group 5 received repeated oral gavage once daily for 7 consecutive days. Animals in groups 1, 2, 3, and 4 were fasted overnight prior to administration on Day 1 and food was restored 4 h after administration. Animals in group 5 were fasted overnight prior to administration on Day 7, and food was restored 4 h after administration. | | | | | | | | |

On day 1 of the test, the animals in group 1 were subjected to single intravenous injection of the inventive sample via the tail vein; the animals in groups 2, 3, and 4 were each subjected to single gavage of the inventive sample at a dosing volume of 5 mL/kg; the animals in group 5 were subjected to gavage of the inventive sample once daily for seven consecutive days. The animals were weighed before administration, and the administration volume was calculated according to the body weight.

4. Sample collection:
After each formulation was prepared, two samples of the intravenous bolus injection (iv) administration solution (top and bottom layers) and three samples of the gavage solution (top, middle, and bottom layers) were collected. The samples were analyzed by using HPLC-UV to investigate the accuracy of the formulation concentration.

The sample collection schedule is shown in Table 15.

**Table 15 Sample collection schedule for intravenous bolus injection and gavage**

| Group | Animal No. | | Sample time point (h) |
|---|---|---|---|
| | Male | Female | |
| 1 | R01, R02, R03 | R04, R05, R06 | Before administration and 0.083 h, 0.25 h, 0.5 h, 1 h, 2 h, 4 h, 6 h, 8 h and 24 h after administration |
| 2 | R07, R08, R09 | R10, R11, R12 | Before administration and 0.25 h, 0.75 h, 1.5 h, 4 h, 6 h, 8 h, 10 h, 12 h, 14 h, 24 h, 36 h and 48 h after administration |
| 3 | R13, R14, R15 | R16, R17, R18 | Before administration and 0.25 h, 0.75 h, 1.5 h, 4 h, 6 h, 8 h, 10 h, 12 h, 14 h, 24 h, 36 h and 48 h after administration |
| 4 | R19, R20, R21 | R22, R23, R24 | Before administration and 0.25 h, 0.75 h, 1.5 h, 4 h, 6 h, 8 h, 10 h, 12 h, 14 h, 24 h, 36 h, 48 h and 72 h after administration |

(continued)

| Group | Animal No. | | Sample time point (h) |
|---|---|---|---|
| | Male | Female | |
| 5 | R25, R26, R27 | R28, R29, R30 | Before administration and 0.25 h, 0.75 h, 1.5 h, 4 h, 6 h, 8 h, 10 h, 12 h, 14 h, 24 h, 36 h, 48 h after administration on day 7, and before administration on days 3, 4, 5 and 6 |

Whole blood samples (about 0.16 mL for groups 1, 2, 3 and 4; for group 5, about 0.08 mL before administration on days 3, 4, 5 and 6, and about 0.17 mL before administration and 0.25 h, 0.75 h, 1.5 h, 4 h, 6 h, 8 h, 10 h, 12 h, 14 h, 24 h, 36 h and 48 h after administration on day 7) were collected at the specified time points via jugular vein puncture (or other appropriate blood collection sites). The actual blood collection time was recorded in the test records. The acceptable error for blood collection time points is ± 1 min for time points within 1 h after administration, and ± 5% of the theoretical time for other time points.

All blood samples were transferred to commercial tubes containing K2-EDTA after collection, and placed on wet ice. Within 60 min after blood collection, the samples were centrifuged at 3200×g for 10 min at about 4 °C. The supernatant plasma was then aspirated, quickly placed in dry ice, and then stored at -60 °C or below for LC-MS/MS analysis.

5. Sample analysis and result discussion:
The concentration of the inventive sample in plasma was determined by using validated high-performance liquid chromatography-tandem mass spectrometry (LC-MS/MS).

The retention times of the compound and internal standard, chromatogram acquisition and chromatogram integration were processed using the software Analyst (Applied Biosystems), and the data statistics were processed using the software Watson LIMS (Thermo Fisher Scientific) or Analyst (SCIEX). The concentration of the analyte in the samples is in unit of ng/mL, with three significant digits retained. For all values expressed as percentages (e.g., % deviation, % coefficient of variation, etc.), one decimal place was retained.

Table 16 shows the results of intravenous bolus injection and gavage, and FIG. 9 shows the mean plasma drug concentration-time curves in male and female SD rats after single gavage of 10 mg/kg, 30 mg/kg and 100 mg/kg of the crystal form A of the compound of formula I.

**Table 16 Results of intravenous bolus injection and gavage**

| Group | 1 | | 2 | | 3 | | 4 | | 5 (day 7) | |
|---|---|---|---|---|---|---|---|---|---|---|
| Route of administration | Intravenous bolus injection | | Gavage | | Gavage | | Gavage | | Gavage | |
| Dose (mg/kg) | 3 | | 10 | | 30 | | 100 | | 30 | |
| Pharmacokinetic parameter | Male | Female | Male | Female | Male | Female | Male | Female | Male | Female |
| $C_0$ or $C_{max}$ (ng/mL) | 943 | 1410 | 280 | 261 | 920 | 1270 | 1650 | 2840 | 2170 | 966 |
| $T_{max}$ (h) | -- | -- | 5.33 | 8.00 | 4.67 | 6.67 | 4.67 | 6.67 | 4.00 | 6.67 |
| Cl (mL/min/kg) | 72.7 | 38.8 | -- | -- | -- | -- | -- | -- | -- | -- |
| $Vd_{ss}$ (L/kg) | 23.0 | 12.5 | -- | -- | -- | -- | -- | -- | -- | -- |
| $AUC_{0-24}$ (ng·h/mL) | 641 | 1220 | 122 0 | 1570 | 3930 | 6610 | 8800 | 17800 | 8740 | 5840 |
| $AUC_{0-last}$ (ng·h/mL) | 641 | 1220 | 124 0 | 1660 | 4160 | 7220 | 9730 | 19700 | 9060 | 6350 |
| $AUC_{0-inf}$ (ng·h/mL) | 692 | 1310 | 149 0 | 1710 | 4190 | 7420 | 1060 0 | 20200 | 9350 | 6750 |
| $T_{1/2}$ (h) | 13.0 | 10.8 | 5.79 | 12.3 | 9.51 | 14.6 | 14.6 | 24.9 | 16.7 | 17.3 |

(continued)

| Group | 1 | | 2 | | 3 | | 4 | | 5 (day 7) | |
|---|---|---|---|---|---|---|---|---|---|---|
| Bioavailability (%)[a] | -- | -- | 58.0 | 40.8 | 64.9 | 59.2 | 45.5 | 48.4 | -- | -- |
| Note: [a] represents Bioavailability was calculated from $AUC_{0\text{-last}}$ and the theoretical dose;represents Not applicable. | | | | | | | | | | |

It can be seen from the above results that after single intravenous bolus injection of 3 mg/kg of the inventive sample in male and female SD rats, the plasma clearance (Cl) values are 72.7 mL/min/kg and 38.8 mL/min/kg, respectively, the steady-state apparent volume of distribution ($Vd_{ss}$) values are 23.0 L/kg and 12.5 L/kg, respectively, and the elimination half-life ($T_{1/2}$) values are 13.0 h and 10.8 h, respectively; the area under the plasma concentration-time curve from point 0 to the last quantifiable time point ($AUC_{0\text{-last}}$) values are 641 ng·h/mL and 1,220 ng·h/mL, respectively.

After single gavage of 10 mg/kg, 30 mg/kg and 100 mg/kg of the inventive sample in male SD rats, the peak concentrations ($C_{max}$) are 280 ng/mL, 920 ng/mL and 1,650 ng/mL, respectively, the times to peak ($T_{max}$) are at 5.33 h, 4.67 h and 4.67 h, respectively; $AUC_{0\text{-last}}$ values are 1,240 ng·h/mL, 4,160 ng·h/mL and 9,730 ng·h/mL, respectively. The bioavailability of the drug in the 10 mg/kg gavage group is 58.0%.

After single gavage of 10 mg/kg, 30 mg/kg and 100 mg/kg of the inventive sample in female SD rats, the peak concentrations ($C_{max}$) are 261 ng/mL, 1,270 ng/mL and 2,840 ng/mL, respectively, the times to peak ($T_{max}$) are at 8.00 h, 6.67 h and 6.67 h, respectively; $AUC_{0\text{-last}}$ values are 1,660 ng·h/mL, 7,220 ng·h/mL and 19,700 ng·h/mL, respectively. The bioavailability of the drug in the 10 mg/kg gavage group is 40.8%.

Except for the 100 mg/kg gavage group where a gender difference was observed in the systemic exposure ($AUC_{0\text{-last}}$) of the inventive sample, no significant gender differences were observed in the systemic exposure ($AUC_{0\text{-last}}$ and $C_0/C_{max}$) in all other groups.

As the intragastric dose increased from 10 mg/kg to 100 mg/kg, the systemic exposure ($AUC_{0\text{-last}}$ and $C_{max}$) in male and female SD rats substantially increased in a dose-proportional manner.

After gavage of 30 mg/kg of the inventive sample once daily for seven consecutive days, the systemic exposure ($AUC_{0\text{-last}}$ and $C_{max}$) in female SD rats remained substantially unchanged, with no significant accumulation. In contrast, the systemic exposure ($AUC_{0\text{-last}}$ and $C_{max}$) in male SD rats showed accumulation in male SD rats, with accumulation indices (day 7/day 1) of 2.18 for $AUC_{0\text{-last}}$ and of 2.36 for $C_{max}$, respectively.

The foregoing descriptions are merely preferred examples of the present disclosure, and it should be noted that for those of ordinary skill in the art, without departing from the principles of the present disclosure, some improvements and refinement may also be made, which should also be considered as the scope of protection of the present disclosure.

## Claims

1. A crystal form of a neuroactive steroid derivative, having characteristic diffraction peaks expressed as 2θ angles in an X-ray powder diffraction pattern obtained by using Cu-Kα radiation, comprising 11.450 ± 0.200°, 15.724 ± 0.200°, 19.040 ± 0.200°, 22.955 ± 0.200° and 24.975 ± 0.200°,
wherein the neuroactive steroid derivative has a structure represented by formula I:

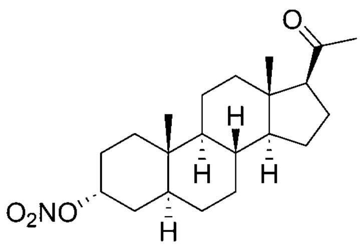

formula I.

2. The crystal form of the neuroactive steroid derivative as claimed in claim 1, wherein the characteristic diffraction peaks further comprise one or more selected from the group consisting of: expressed as 2θ angles, 7.843 ± 0.200°, 9.643 ± 0.200°, 12.338 ± 0.200°, 13.384 ± 0.200°, 14.210 ± 0.200°, 15.032 ± 0.200°, 16.498 ± 0.200°, 17.148 ± 0.200°, 17.802 ± 0.200°, 18.739 ± 0.200°, 19.313 ± 0.200°, 19.726 ± 0.200°, 20.276 ± 0.200°, 22.464 ± 0.200°, 23.558 ± 0.200°, 25.897 ± 0.200°, 26.445 ± 0.200°, 26.914 ± 0.200°, 27.202 ± 0.200°, 27.964 ± 0.200°, 28.781 ± 0.200°, 29.102 ± 0.200°, 29.451 ± 0.200°, 29.760 ± 0.200°, 31.706 ± 0.200°, 32.133 ± 0.200°, 32.616 ± 0.200°, 32.922 ± 0.200°, 33.952 ± 0.200°, 34.662 ± 0.200°, 36.355 ± 0.200°, 36.778 ± 0.200°, 37.511 ± 0.200°, 38.559 ± 0.200°, and

39.073 ± 0.200°.

3. The crystal form of the neuroactive steroid derivative as claimed in claim 1 or 2, wherein in the X-ray powder diffraction pattern, there are the characteristic diffraction peaks at 20 angles: 7.843 ± 0.200°, 11.450 ± 0.200°, 15.724 ± 0.200°, 18.739 ± 0.200°, 19.040 ± 0.200°, 19.726 ± 0.200°, 22.955 ± 0.200°, and 24.975 ± 0.200°.

4. The crystal form of the neuroactive steroid derivative as claimed in claim 1 or 2, wherein in the X-ray powder diffraction pattern, there are the characteristic diffraction peaks at 2θ angles: 7.843 ± 0.200°, 11.450 ± 0.200°, 13.384 ± 0.200°, 15.724 ± 0.200°, 16.498 ± 0.200°, 17.148 ± 0.200°, 18.739 ± 0.200°, 19.040 ± 0.200°, 19.726 ± 0.200°, 20.276 ± 0.200°, 22.955 ± 0.200°, 23.558 ± 0.200°, 24.975 ± 0.200°, 28.781 ± 0.200°, and 29.102 ± 0.200°.

5. The crystal form of the neuroactive steroid derivative as claimed in claim 1 or 2, wherein in the X-ray powder diffraction pattern, there are the characteristic diffraction peaks at 2θ angles: 7.843 ± 0.200°, 11.450 ± 0.200°, 13.384 ± 0.200°, 14.210 ± 0.200°, 15.724 ± 0.200°, 16.498 ± 0.200°, 17.148 ± 0.200°, 18.739 ± 0.200°, 19.040 ± 0.200°, 19.313 ± 0.200°, 19.726 ± 0.200°, 22.955 ± 0.200°, 23.558 ± 0.200°, 24.975 ± 0.200°, 29.102 ± 0.200°, 36.355 ± 0.200°, and 37.511 ± 0.200°.

6. The crystal form of the neuroactive steroid derivative as claimed in claim 1 or 2, wherein in the X-ray powder diffraction pattern, there are the characteristic diffraction peaks at 20 angles: 7.843 ± 0.200°, 11.450 ± 0.200°, 13.384 ± 0.200°, 14.210 ± 0.200°, 15.724 ± 0.200°, 16.498 ± 0.200°, 17.148 ± 0.200°, 18.739 ± 0.200°, 19.040 ± 0.200°, 19.313 ± 0.200°, 19.726 ± 0.200°, 20.276 ± 0.200°, 22.955 ± 0.200°, 23.558 ± 0.200°, 24.975 ± 0.200°, 28.781 ± 0.200°, 29.102 ± 0.200°, 36.355 ± 0.200°, and 37.511 ± 0.200°.

7. The crystal form of the neuroactive steroid derivative as claimed in claim 1 or 2, wherein in the X-ray powder diffraction pattern, there are the characteristic diffraction peaks at 20 angles: 7.843 ± 0.200°, 9.643 ± 0.200°, 11.450 ± 0.200°, 12.338 ± 0.200°, 13.384 ± 0.200°, 14.210 ± 0.200°, 15.032 ± 0.200°, 15.724 ± 0.200°, 16.498 ± 0.200°, 17.148 ± 0.200°, 17.802 ± 0.200°, 18.739 ± 0.200°, 19.040 ± 0.200°, 19.313 ± 0.200°, 19.726 ± 0.200°, 20.276 ± 0.200°, 22.464 ± 0.200°, 22.955 ± 0.200°, 23.558 ± 0.200°, 24.975 ± 0.200°, 25.897 ± 0.200°, 26.445 ± 0.200°, 26.914 ± 0.200°, 27.202 ± 0.200°, 27.964 ± 0.200°, 28.781 ± 0.200°, 29.102 ± 0.200°, 29.451 ± 0.200°, 29.760 ± 0.200°, 31.706 ± 0.200°, 32.133 ± 0.200°, 32.616 ± 0.200°, 32.922 ± 0.200°, 33.952 ± 0.200°, 34.662 ± 0.200°, 36.355 ± 0.200°, 36.778 ± 0.200°, 37.511 ± 0.200°, 38.559 ± 0.200°, and 39.073 ± 0.200°.

8. The crystal form of the neuroactive steroid derivative as claimed in claim 1 or 2, wherein a differential scanning calorimetry pattern of the crystal form of the neuroactive steroid derivative has an endothermic peak at 139.9 °C ± 3 °C; and an onset value of the absorption peak is at 138.5 °C ± 3 °C.

9. The crystal form of the neuroactive steroid derivative as claimed in claim 1 or 2, wherein a thermogravimetric analysis pattern of the crystal form of the neuroactive steroid derivative shows a weight loss of ± 0.5% at 159.4 °C ± 3 °C.

10. The crystal form of the neuroactive steroid derivative as claimed in claim 1 or 2, wherein the crystal form of the neuroactive steroid derivative is of an orthorhombic crystal system, with a space group of $P2_12_12_1$, and
the crystal form of the neuroactive steroid derivative has unit cell parameters: a = 7.40840(10)Å, b = 14.3545(2)Å, c = 18.1883(3)Å, α = 90°, β = 90°, γ = 90°; a unit cell volume of 1934.21(5)Å$^3$; and Z = 4.

11. The crystal form of the neuroactive steroid derivative as claimed in claim 1 or 2, wherein the crystal form of the neuroactive steroid derivative has a particle size D90 of 240-530 μm, a D50 of 105-270 μm, and a D10 of 20-65 μm.

12. The crystal form of the neuroactive steroid derivative as claimed in claim 1 or 2, wherein the crystal form of the neuroactive steroid derivative has an angle of repose of 39.2° and a Carr index of 14%.

13. Use of the crystal form of the neuroactive steroid derivative as claimed in any one of claims 1-12 in preparation of a drug for treating emotional disorders, psychiatric diseases, neurodegenerative diseases, or neuropathic pain.

14. The use as claimed in claim 13, wherein the emotional disorders comprise generalized anxiety disorder, panic disorder, obsessive-compulsive disorder, post-traumatic stress disorder, social anxiety disorder, premenstrual dysphoric disorder, major depression, or postpartum depression.

15. The use as claimed in claim 13, wherein the psychiatric diseases comprise epilepsy, convulsion, or insomnia.

**16.** The use as claimed in claim 13, wherein the neurodegenerative diseases comprise Alzheimer's disease, Parkinson's disease, multiple sclerosis, or Niemann-Pick disease type C.

**17.** The use as claimed in claim 13, wherein the neuropathic pain comprises menstrual and postmenopausal migraine, diabetic peripheral neuralgia, chemotherapy-induced pain, or sciatica.

**18.** The use as claimed in any one of claims 13-17, wherein the drug comprises an active ingredient and a pharmaceutically acceptable carrier, wherein the active ingredient is the crystal form of the neuroactive steroid derivative; and the pharmaceutically acceptable carrier is a solid carrier or a liquid carrier.

**19.** The use as claimed in claim 18, wherein the solid carrier comprises one or more selected from the group consisting of cellulose, glucose, lactose, mannitol, magnesium stearate, magnesium carbonate, sodium carbonate, sodium saccharin, sucrose, dextrin, talc, starch, pectin, gelatin, tragacanth gum, acacia gum, sodium alginate, paraben, methylcellulose, sodium carboxymethylcellulose, low-melting wax, and cocoa butter.

**20.** The use as claimed in claim 18, wherein the liquid carrier comprises one or more selected from the group consisting of water, ethanol, a polyol, a vegetable oil, glyceride, agar, pyrogen-free water, isotonic saline, and Ringer's solution.

**21.** The use as claimed in claim 20, wherein the polyol comprises one or more selected from the group consisting of glycerol, propylene glycol, and liquid polyethylene glycol; and the vegetable oil comprises one or more selected from the group consisting of peanut oil, cottonseed oil, safflower oil, sesame oil, olive oil, corn oil, and soybean oil.

**22.** The use as claimed in any one of claims 13-17, wherein an administration route of the drug comprises oral administration, intrapulmonary administration, intranasal administration, or transdermal administration.

**23.** The use as claimed in claim 22, wherein a dosage form of the drug comprises a tablet, a lozenge, a buccal tablet, a suspension, a dispersible powder, a dispersible granule, an emulsion, a hard capsule, a soft capsule, a syrup, an elixir, a solution, a spray, an aerosol or dry powder formulation, a nasal drop or a nasal spray.

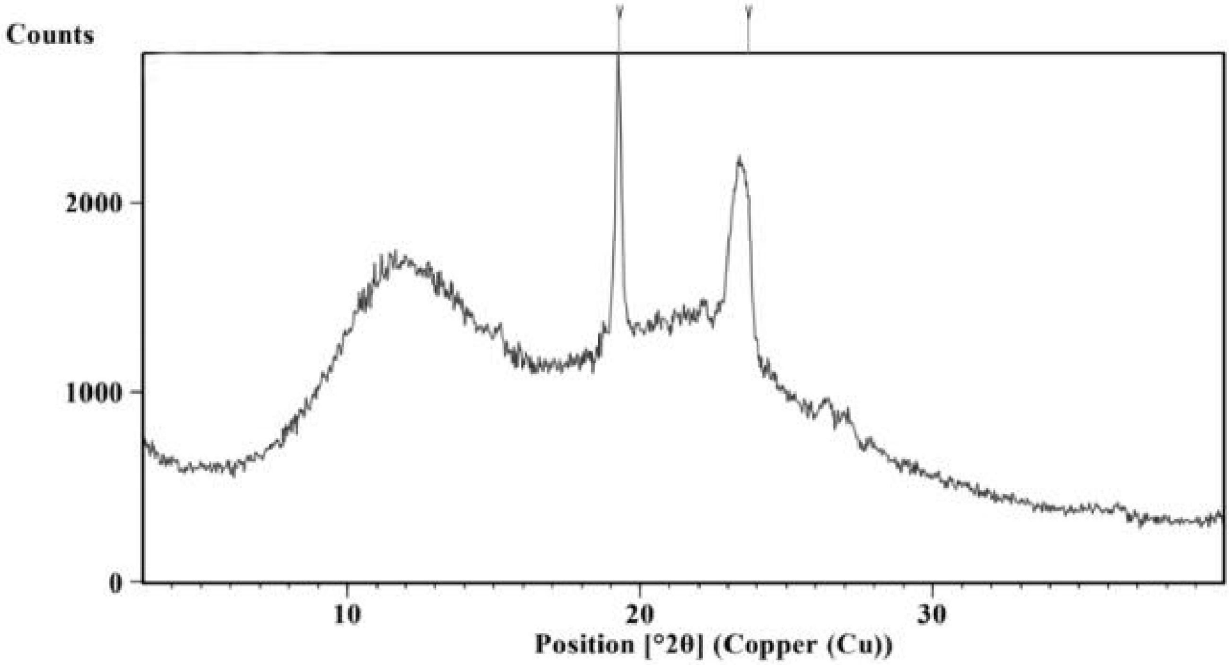
Counts
2000
1000
0
10
20
30
Position [°2θ] (Copper (Cu))

FIG. 1

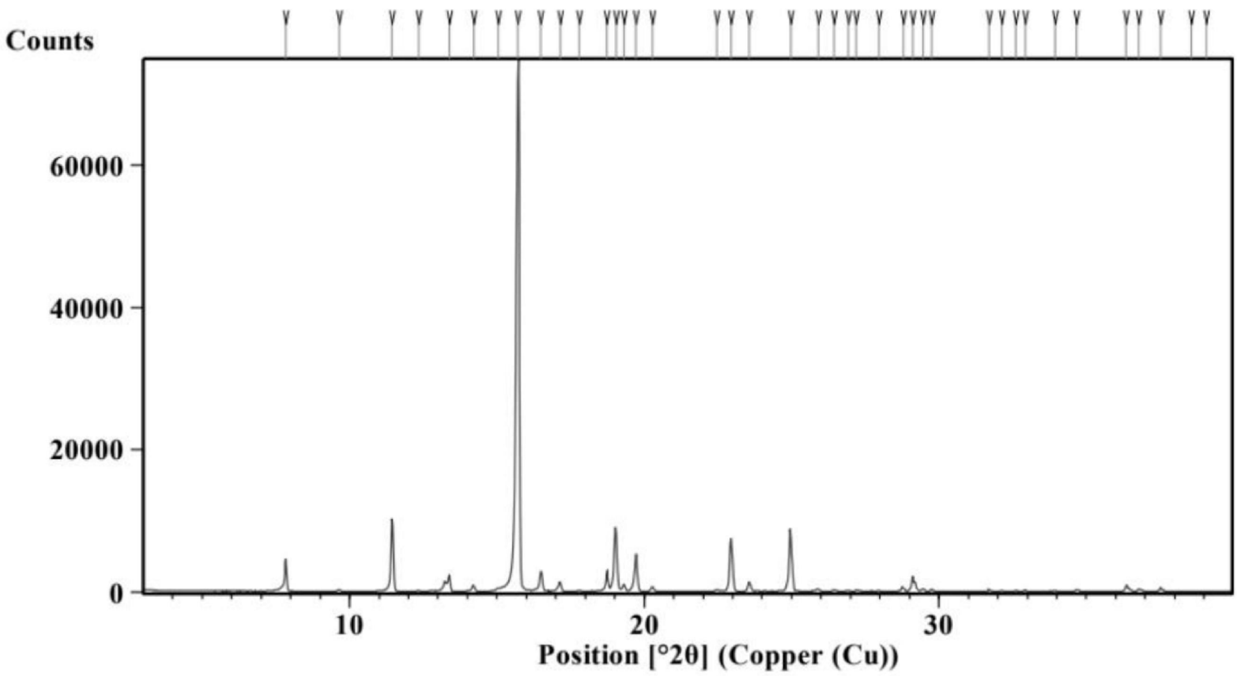
Counts
60000
40000
20000
0
10
20
30
Position [°2θ] (Copper (Cu))

FIG. 2

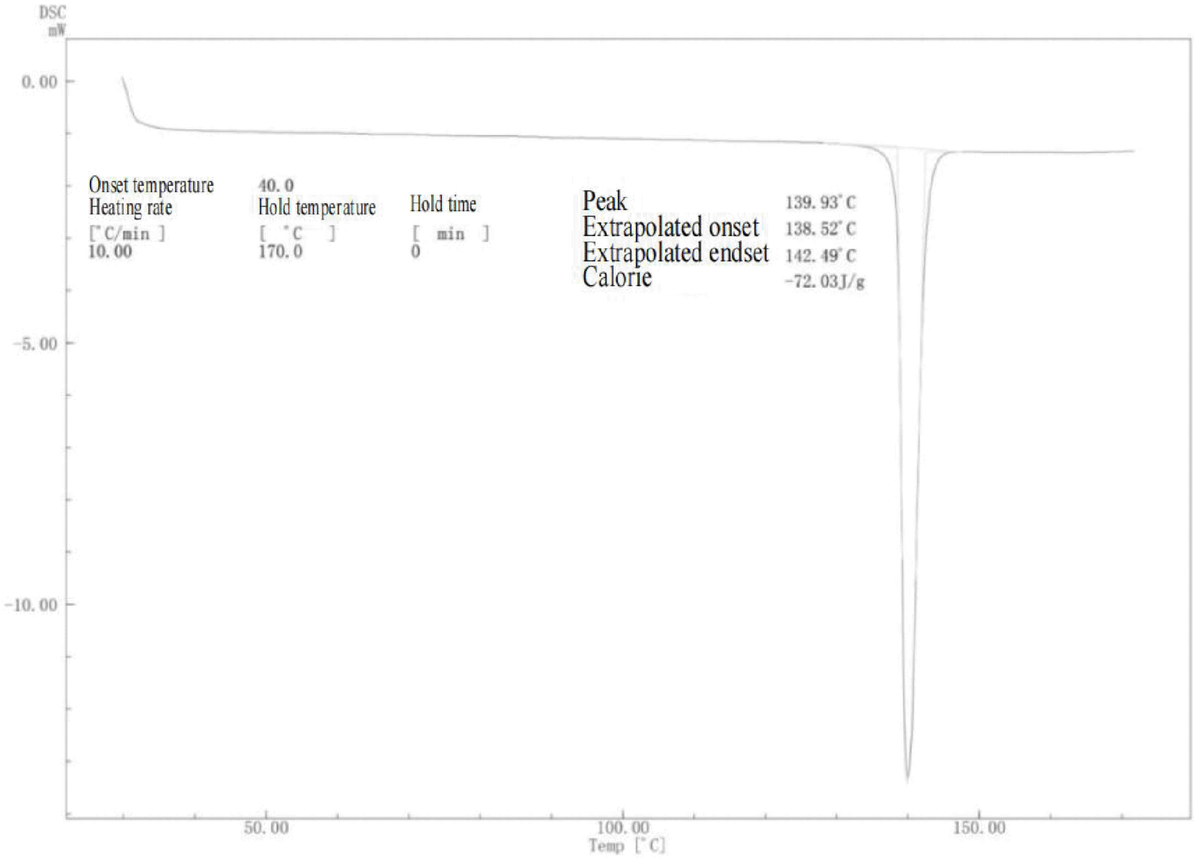
DSC
mW
0.00
-5.00
-10.00
Onset temperature 40.0
Heating rate
[°C/min ]
10.00
Hold temperature
[ °C ]
170.0
Hold time
[ min ]
0
Peak 139.93°C
Extrapolated onset 138.52°C
Extrapolated endset 142.49°C
Calorie -72.03J/g
50.00
100.00
150.00
Temp [°C]

FIG. 3

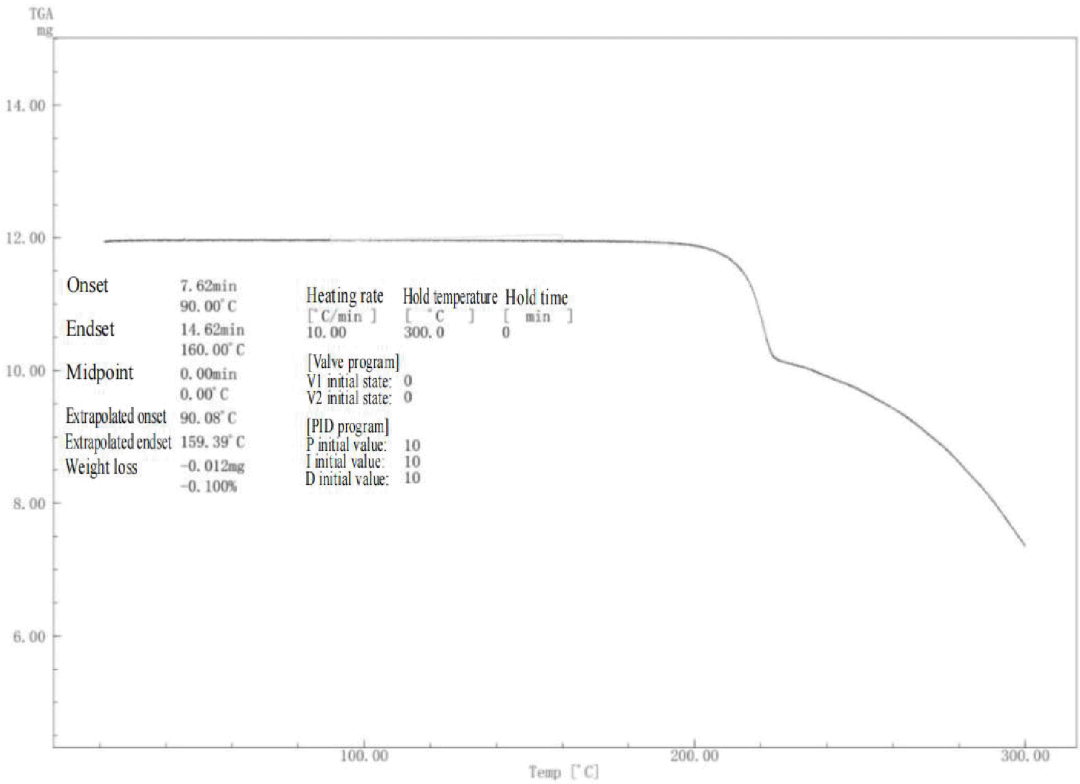
TGA
mg
14.00
12.00
10.00
8.00
6.00
Onset 7.62min
90.00°C
Endset 14.62min
160.00°C
Midpoint 0.00min
0.00°C
Extrapolated onset 90.08°C
Extrapolated endset 159.39°C
Weight loss -0.012mg
-0.100%
Heating rate
[°C/min ]
10.00
Hold temperature
[ °C ]
300.0
Hold time
[ min ]
0
[Valve program]
V1 initial state: 0
V2 initial state: 0
[PID program]
P initial value: 10
I initial value: 10
D initial value: 10
100.00
200.00
300.00
Temp [°C]

FIG. 4

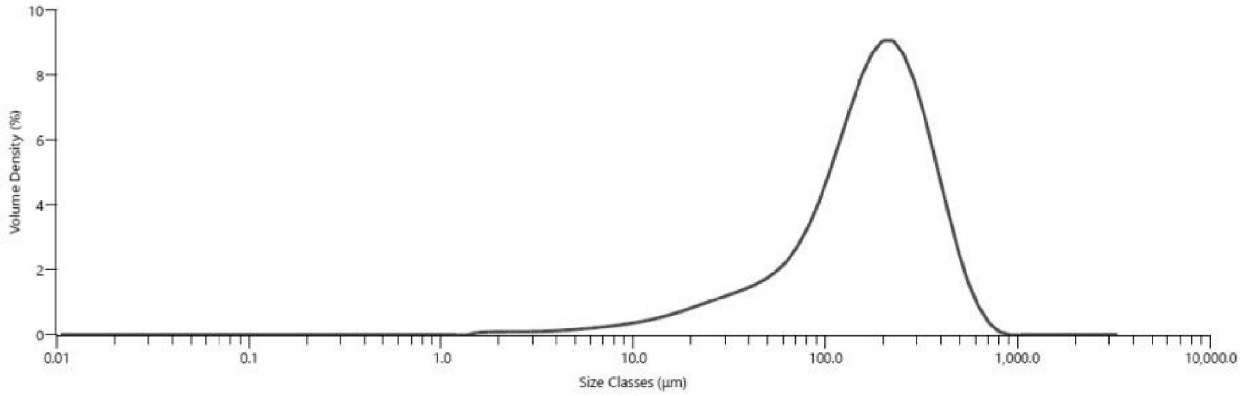
10
8
6
4
2
0
Volume Density (%)
0.01
0.1
1.0
10.0
100.0
1,000.0
10,000.0
Size Classes (μm)

FIG. 5

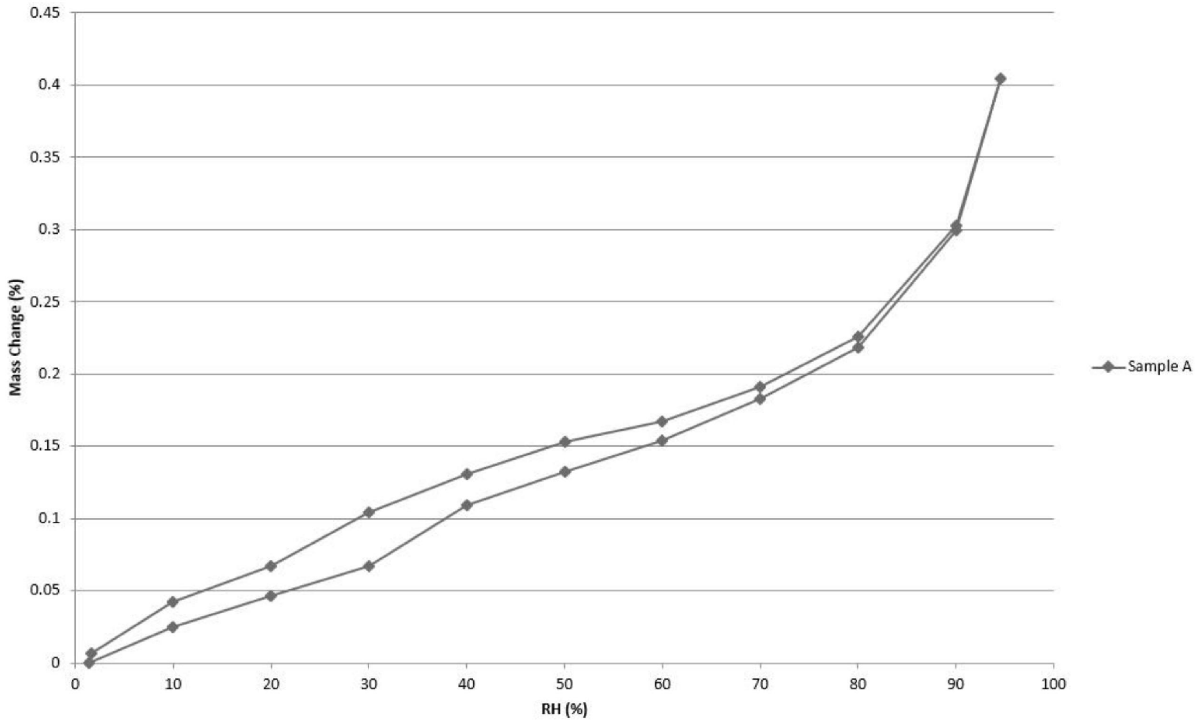
0.45
0.4
0.35
0.3
0.25
0.2
0.15
0.1
0.05
0
Mass Change (%)
0
10
20
30
40
50
60
70
80
90
100
RH (%)
Sample A

FIG. 6

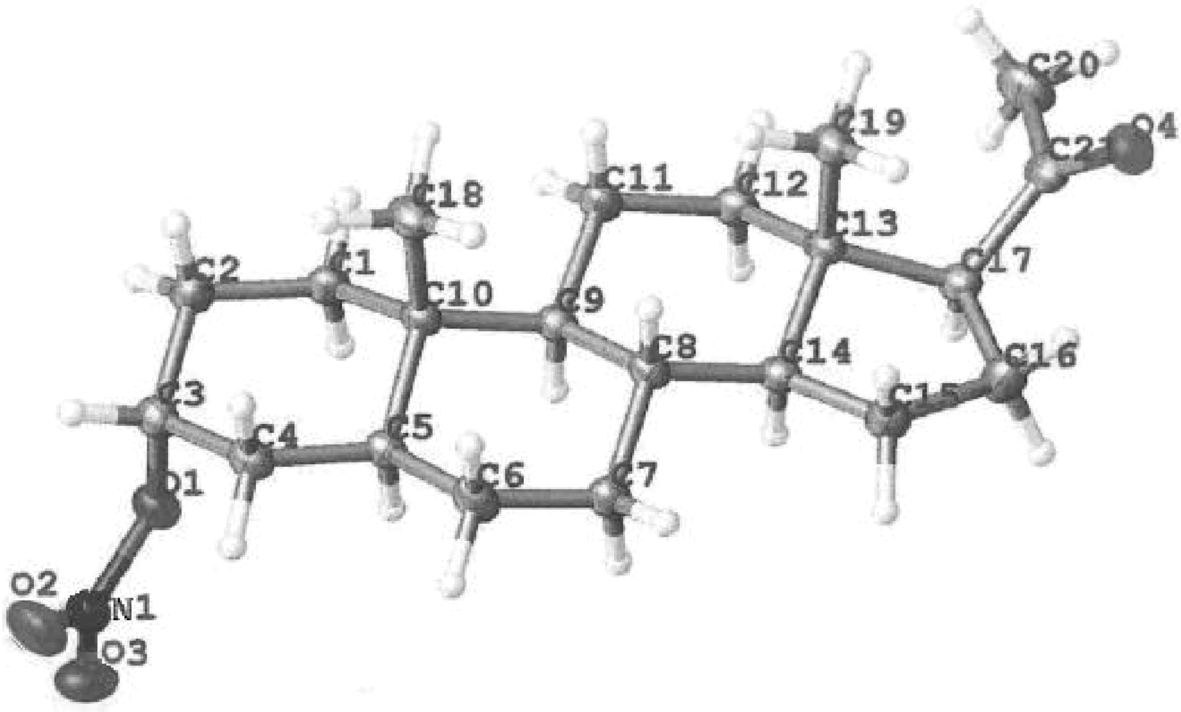

FIG. 7

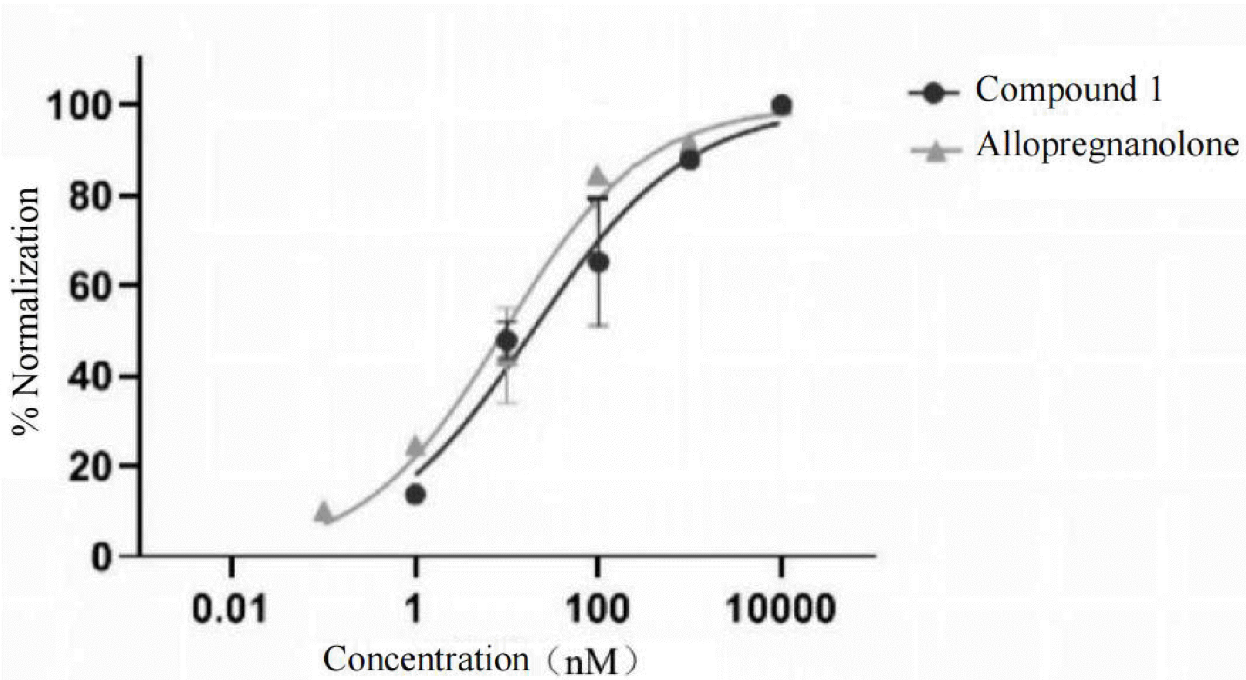
% Normalization
0
20
40
60
80
100
0.01
1
100
10000
Concentration（nM）
Compound 1
Allopregnanolone

FIG. 8

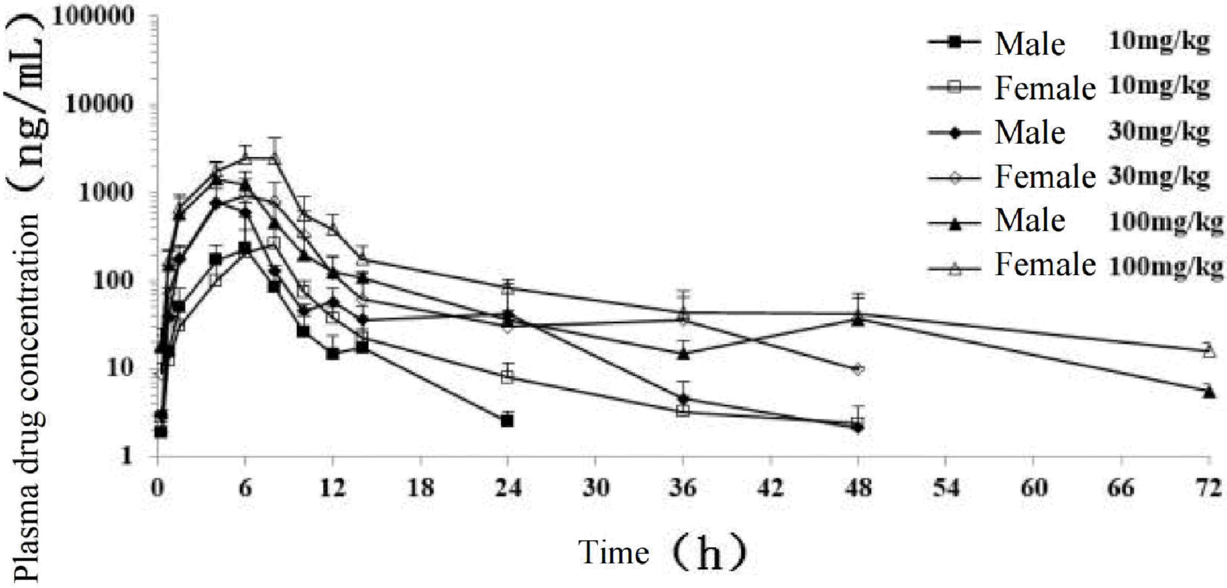
Plasma drug concentration（ng/mL）
100000
10000
1000
100
10
1
0
6
12
18
24
30
36
42
48
54
60
66
72
Time（h）
Male 10mg/kg
Female 10mg/kg
Male 30mg/kg
Female 30mg/kg
Male 100mg/kg
Female 100mg/kg

FIG. 9

**INTERNATIONAL SEARCH REPORT**

International application No.
**PCT/CN2025/118667**

**A. CLASSIFICATION OF SUBJECT MATTER**

C07J41/00(2006.01)i; A61K31/57(2006.01)i; A61P25/00(2006.01)i; A61P25/04(2006.01)i; A61P25/06(2006.01)i; A61P25/08(2006.01)i; A61P25/20(2006.01)i; A61P25/22(2006.01)i; A61P25/24(2006.01)i; A61P25/28(2006.01)i; A61P25/16(2006.01)i

According to International Patent Classification (IPC) or to both national classification and IPC

**B. FIELDS SEARCHED**

Minimum documentation searched (classification system followed by classification symbols)

IPC: C07J, A61K, A61P

Documentation searched other than minimum documentation to the extent that such documents are included in the fields searched

Electronic data base consulted during the international search (name of data base and, where practicable, search terms used)

CNTXT, ENTXT, ENTXTC, DWPI, CJFD, REGISTRY(STN), CAPLUS(STN), MARPAT(STN), Web of Science, CNKI, 万方, WANFANG, 百度学术, BAIDU SCHOLAR: 明瑞制药, 华睿鼎信, 安明, 张旭, 苏胜, 王岩, 况斌, 晶型, 神经活性类固醇, 硝酸酯, 抑郁, crystal, NAS, neuroactive steroid, nitrat+, depression, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| PX | CN 119409754 A (BEIJING HORICIN BIOTECHNOLOGY CO., LTD.) 11 February 2025 (2025-02-11)<br>claims 1-10 | 1-23 |
| X | CN 117801047 A (BEIJING HORICIN BIOTECHNOLOGY CO., LTD.) 02 April 2024 (2024-04-02)<br>claims 7-9, and description, paragraphs 0057-0066 and 0076-0079 | 1-23 |
| A | BOROVSKA, J. et al. "Access of Inhibitory Neurosteroids to the NMDA Receptor" *British Journal of Pharmacology*, Vol. 166, 31 December 2012 (2012-12-31),<br>pages 1069-1083 | 1-23 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 November 2025** | **14 November 2025** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2025/118667**

| Patent document cited in search report | | | Publication date (day/month/year) | Patent family member(s) | | | Publication date (day/month/year) |
|---|---|---|---|---|---|---|---|
| CN | 119409754 | A | 11 February 2025 | None | | | |
| CN | 117801047 | A | 02 April 2024 | WO | 2024153267 | A2 | 25 July 2024 |
| | | | | WO | 2024153267 | A3 | 12 September 2024 |
| | | | | EP | 4635968 | A2 | 22 October 2025 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202411536243


### Non-patent literature cited in the description


- *CHEMICAL ABSTRACTS*, 516-54-1